(19) Europäisches Patentamt
European Patent Office
Office européen des brevets

(11) **EP 1 922 989 A2**

(12) **EUROPÄISCHE PATENTANMELDUNG**

(43) Veröffentlichungstag:
**21.05.2008 Patentblatt 2008/21**

(51) Int Cl.:
*A61B 5/00* *(2006.01)*

(21) Anmeldenummer: **07022254.2**

(22) Anmeldetag: **15.11.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **15.11.2006 DE 102006053786**
**27.09.2007 DE 102007046295**

(71) Anmelder: **Buschmann, Johannes P.**
**81669 München (DE)**

(72) Erfinder: **Buschmann, Johannes P.**
**81669 München (DE)**

(54) **Verfahren und Vorrichtung für die kontinuierliche und mobile Messung verschiedener Vitalparameter im äußeren Gehörgang**

(57) Die Erfindung beschreibt ein Verfahren und eine Vorrichtung zur kontinuierlichen, mobilen nicht-invasiven und ästhetisch unauffälligen Messung von wichtigen Vitalparametern, insbesondere des Blutdrucks und des EKGs, der Körper(kern)temperatur, der arteriellen Sauerstoffsättigung, der Herzfrequenz, der Atemfrequenz (mittels Pulsoximetrie) sowie der Bestimmung relativer bzw. absoluter Stoffkonzentrationen in (arteriellem) Blut oder Gewebe und schließlich mechanischer Parameter. Der Messort und der Sitz der Sensoren ist der (proximale) Gehörgang. Eine elegante Aufhängung der Sensoren im äußeren Gehörgang bewirkt eine stabile und bewegungsarme Positionierung auch und gerade unter mobilen Bedingungen und unauffällig im Alltag.

Die Sensorik steht in Verbindung mit einer kleinen Auswerteeinheit hinter der Ohrmuschel, die Energiezellen und Elektronik zur Messung und Aufbereitung der Signale und gegebenenfalls Einrichtungen zum drahtlosen Weiterleiten der Signale z.B. mittels Mobilfunk enthält.

So werden wichtige physiologische Parameter zugänglich für die Langzeitdiagnostik, für ambulante Patienten, für ein Monitoring während einer Rehabilitation, aber auch zur Überwachung der Gesundheit im Alltag, für fundierte Informationen beim Sport und während des Trainings, für eine Erhöhung der Sicherheit gefährdeter Personen bzw. von Menschen mit gefährlichen Berufen oder mit riskanten Hobbys.

Figur 1

EP 1 922 989 A2

**Beschreibung**

### I. Gebiet der Erfindung

Die Erfindung betrifft ein Verfahren sowie eine Vorrichtung zur nicht-invasiven

Messung wichtiger physiologischer Parameter bei Mensch oder Tier insbesondere kontinuierlich und unter mobilen Bedingungen. Dazu zählen insbesondere die Messung der Körper(kern)temperatur, gewebsoptischer Parameter wie der arteriellen Sauerstoffsättigung, der Herzfrequenz und der Atemfrequenz, der Konzentrationen bestimmter Stoffe in Blut und/oder Gewebe ohne eine Gewinnung von Körperflüssigkeiten, aber auch die Messung des Blutdrucks, des EKGs, sowie mechanische Parameter wie Lage, Position und Beschleunigung. Als Messort wird der Gehörgang verwendet.

### II. Hintergrund der Erfindung

Die moderne Medizin zeigt die Tendenz zu weniger invasiven Eingriffen (minimal invasive surgery) und zu immer kürzeren Liegezeiten. Viele diagnostische und therapeutische Maßnahmen, viele operative Eingriffe, die früher stationär durchgeführt wurden, werden heute ambulant ausgeführt oder doch wenigstens in deutlich kürzeren Hospitalisierungszeiten. Dieser Trend sollte konsequenterweise von Monitoring-Technologien flankiert werden, die es möglich machen, den ambulanten Patienten (outpatient) mehr oder weniger kontinuierlich zu überwachen, um seine Gesundheit sicherzustellen und jedwede Gefährdung auch außerhalb des Krankenhauses zu erkennen. Viele Gefährdungen sind kontinuierlicher Natur und begleiten daher den Alltag wie z.B. Diabetes, Allergien,

Hypertonie, Chronic Obstructive Pulmonary Disease (COPD), Fettstoffwechselstörungen usw.

Aber nicht nur der Patient, auch der gesunde, mobile Mensch wird zunehmend gesundheitsbewusster und sucht einerseits seine Gesundheit durch diverse Aktivitäten zu erhalten und zu verbessern z.B. durch Training, Besuch von Fitness-Studios, aktive Freizeitgestaltung, Diätetik usw. andererseits möchte er auch informiert werden, ob wichtige (prognostische) Parameter in Ordnung sind, insbesondere ob die gesundheitsfördernden Maßnahmen auch tatsächlich Wirkung zeigen, ob z.B. ein avisierter Trainingseffekt objektivierbar ist oder ähnliche Fragestellungen.

Schließlich sind auch bestimmte Berufsgruppen, z.B. Piloten, Feuerwehrleute, Taucher, Kühllageristen, Bergretter, Hochofenarbeiter, Glasbläser, usw. potentiell gefährdet durch Sauerstoffmangel, Feinstaub, durch Unterkühlung oder Hyperthermie. Diese Berufsgruppen wünschen diese Gefährdung durch die Bestimmung relevanter physiologischer Parameter zu quantifizieren, um die Gefährdung einschätzen oder, um sie begrenzen zu können.

Die Entwicklung in der Humansensorik erlaubt heute schon den Zugang zu diagnostisch wichtigen Parametern, wie Lungenfunktion, endtidal $pCO_2$, Blutdruck, Körpertemperatur, Sauerstoffsättigung, Elektrokardiogramm (EKG) und andere mehr. Von ganz wenigen Ausnahmen abgesehen (12-Kanal-Langzeit-EKG oder an die 24-Stunden-Blutdruckmessung) bleiben die meisten physiologischen Parameter nur einer punktuellen Messung oder kurzzeitigen Messverläufen vorbehalten, oder sie sind nur unter klinischen Bedingungen (= Intensivmonitoring) zugänglich. Viele biochemische Parameter bedürfen sogar der Gewinnung von Blutproben.

### III. Stand der Technik

#### Messung der Körper(kern)temperatur

Es gibt eine Reihe von Möglichkeiten zur Messung der Körpertemperatur, denen allen gemeinsam ist, dass meist nur eine Maximaltemperatur gemessen wird und nur eine diskontinuierliche Messung erfolgt. Nur unter den Bedingungen der Intensivmedizin wird die Körpertemperatur als kontinuierliche Messgröße bestimmt.

Prinzipiell kann die Messung der Körpertemperatur an verschiedenen mehr oder weniger geeigneten Orten erfolgen, wobei dann die gemessene Temperatur mehr oder weniger relevant für das ist, was als "Körper(kern)temperatur" bezeichnet wird. Unter Körper(kern)temperatur wird einerseits eine im räumlich-anatomischen Sinne zentrale Temperatur, eine Temperatur der innersten Organe verstanden, die nicht durch unmittelbare äußere Einflüsse gestört ist. Auf der anderen Seite versteht man unter Körper(kern)temperatur eine nahe dem Hypothalamus gemessene Temperatur, weil hier der physiologische Sensor für die Temperaturregelung sitzt und sie somit für den thermischen Haushalt des Organismus die größte Bedeutung hat. Unter diesen Gesichtspunkten ist eine im äußeren Gehörgang gemessene Körper(kern)temperatur eine herausragend relevante Größe.

Es sind im Gehörgang ausschließlich Strahlungssensoren beschrieben, unter der Vorstellung, dass die Strahlung aus dem hinteren Gehörgang die Körper(kern)temperatur repräsentiert. Strahlungssensoren sind aber nicht sehr genau. Bislang werden Messungen der Körpertemperatur am Ohr nur punktuell durchgeführt, d.h. diskontinuierlich.

Die Körper(kern)temperatur ist eine wichtige physiologische Größe zur allgemeinen Überwachung, da sie viele wichtige Zustände des Körpers widerspiegelt. Die Verfügbarkeit des Parameters "kontinuierliche, im Alltag messbare Körper(kern)temperatur", seine praktische Anwendung und vor allem die von diesem Parameter ableitbaren physiologischen Informationen sind heute noch nicht einmal abschätzbar. Es wird sich zeigen, welche Informationen sich anhand dieser Messgröße gewinnen lassen, ebenso, wie sich herausstellte, welche Bedeutung ein Langzeit-EKG hat oder eine 24 Stunden Blutdruckmessung. Im Folgenden werden interessante Anwendungen für eine kontinuierliche Messung der Körper(kern)temperatur skizziert:

Die Körper(kern)temperatur wird durch körperliche Aktivität erhöht, sodass die Körper(kern)temperatur sowohl im beruflichen Umfeld als auch im Sport zur Dosierung körperlicher Aktivitäten verwendet werden kann. Es ist durchaus vorstellbar, dass in naher Zukunft Sportler anhand ihrer Körper(kern)temperatur ausgewechselt werden, z.B. wenn sie eine kritische Temperatur erreicht haben - ausgehend von der Vorstellung, dass eine Überhitzung einen unphysiologischen Zustand bedeutet, der mit einer Abnahme körperlicher Leistungsfähigkeit einhergeht. Die Körper(kern)temperatur hat auch etwas mit dem Grundumsatz zu tun, der wiederum von den Schilddrüsenhormonen beeinflusst wird, aber auch von Adrenalin und vom Wachstumshormon sowie anderen Botenstoffen.

Die Körper(kern)temperatur wird von Gestagenen beeinflusst, die eine Erhöhung der Solltemperatur veranlassen, sodass sich die zweite Zyklushälfte der Frau durch eine Erhöhung der Basaltemperatur erkennen lässt. Damit ist die Körper-(kern)temperatur eine Größe, aus der sich der Eisprung erkennen lässt und damit die sog. "fruchtbaren Tage der Frau".

Die Körper(kern)temperatur ist eine geregelte Größe. Damit entspricht der Istwert normalerweise dem Sollwert. Der Sollwert kann aber beispielsweise durch Pyrogene verstellt werden, sodass Fieber auftritt, im Sinne einer Vergiftung durch Bakterientoxine, beispielsweise bei Infektionskrankheiten. Aber auch allergische Reaktionen können mit Fieber einhergehen, z.B. Abstoßungsreaktionen von Transplantaten, Transfusionsreaktionen, Impfreaktionen, Schlangenbiss und andere Inokulationen tierischer Gifte oder jedweder Antigene.

Der Temperaturregler wird anästhesiert durch viele Stoffe, die das Zentralnervensystem dämpfen, z.B. durch Alkohol oder Schlafmittel in höheren Mengen und durch Narkotika. Dann nähert sich die Körper(kern)temperatur der Umgebungstemperatur an, desto schneller, je geringer die Wärmewiderstände sind. Die Körper(kern)temperatur kann prinzipiell in beide Richtungen entgleisen, eine bedrohliche Situation, die es zu diagnostizieren gilt, um sie behandeln zu können.

Die Körper(kern)temperatur steht des Weiteren in engem Zusammenhang mit der Vigilanz. Unabhängig von rein körperlicher Aktivität verändert sich die Körper-(kern)temperatur als Funktion der Vigilanz. Somit kann die Körper (kern)-temperatur zur Bestimmung der Vigilanz herangezogen werden.

Auch der Schlaf-Wach-Rhythmus verändert die Temperaturregelung: In den REM-Phasen des Schlafs ist die Temperaturregelung reduziert, während sie in den anderen Schlafphasen normal ist.

Von entscheidender Bedeutung ist die Entdeckung von Ermüdungszuständen, insbesondere die Entdeckung oder besser noch die Vorhersage der gefürchteten Sekundenschlaf-Ereignisse beim Autofahren. Es bleibt zu untersuchen, in wie weit die Körper(kern)temperatur oder einer der anderen vorgeschlagenen Parameter sich als Indikator hierfür eignet.

### Messung des Blutdrucks:

Die unblutige, d.h. nicht-invasive Messung des Blutdrucks beruht bislang ausschließlich auf der Verwendung von Manschetten, üblicherweise am Arm, selten am Finger. Diese Blutdruckmanschetten sind im Außenbereich umfangstabil und erlauben das zentripetale Komprimieren des Gewebes bis zur vollständigen Okklusion der im Gewebe verlaufenden Arterien und Arteriolen. Andere Messorte, die eine mobile, nicht-invasive Blutdruckmessung erlauben würden, sind nicht beschrieben.

### EKG:

Die klassischen Orte für die Platzierung von EKG-Elektroden erfolgt an der Thoraxwand. Andere Orte finden beim Erwachsenen keine Anwendung. Beim Feten wird ein EKG an der Kopfhaut abgeleitet, allerdings mit dem mütterlichen Körper / Abdomen als Gegen- bzw. als Referenzelektrode. Ein mobiles EKG ohne Aufkleben von Elektroden bzw. ohne Kleidung, die Elektroden enthält, ist bisher nicht verfügbar

### Messung fotometrischer und optisch-plethysmografischer Größen-Pulsoximetrie:

Die Pulsoximetrie ist ein optisches Verfahren zur Bestimmung der Sauerstoffsättigung über die Fotometrie des Gewebes mit den darin pulsierenden beiden Varianten des arteriellen Hämoglobinfarbstoffs $Hb_{ox}$ und $Hb_{red}$. Sie ist weit verbreitet und bereits hoch miniaturisiert. Eine mobile Pulsoximetrie scheitert derzeit am Messort: der Messort ist fast immer der Finger, andere Messorte sind weniger zuverlässig. So ist eine mobile, im Alltag nicht störende Pulsoximetrie bislang nicht verfügbar, obwohl dafür ein hoher Bedarf besteht.

Ein Zugriff auf die Information Sauerstoffmangel ist in doppelter Weise wichtig: Erstens ist die Funktionalität und Integrität des Gehirns durch Sauerstoffmangel schnell und bedrohlich limitiert. Zweitens erlaubt gerade eine mobile und kontinuierliche Messung der Sauerstoffsättigung einen diagnostischen Blick auf eine im Zunehmen befindliche Zivilisationskrankheit mit hoher Dunkelziffer und oft langer Zeit zwischen Erkrankungsbeginn und Diagnosestellung: die COPD, die chronisch obstruktive Lungenerkrankung. Rauchen, Feinstaub in der Luft oder Allergien stören die Ausatmung und führen langfristig zu COPD, die heute schon für ca. ein Drittel aller Todesfälle verantwortlich ist. Der Parameter Sauerstoffsättigung erlaubt es die reduzierte Lungenfunktion insbesondere unter Belastung zu detektieren. Benutzt man einen Sensor für das Parameter-Bündel Sauerstoffsättigung, Herzfrequenz und Atemfrequenz, so lässt sich die Diagnose COPD unter Umständen schon zu einem Zeitpunkt stellen, an dem die Prognose noch deutlich besser ist.

Der Parameter Sauerstoffsättigung ist zudem immer dann ein Indikator einer Gefährdung, wenn ein verminderter Sauerstoffpartialdruck zu erwarten oder doch wenigstens möglich ist. Berufsgruppen wie Feuerwehrleute, Piloten usw. aber auch Aktivitäten wie Bergsteigen, Tauchen usw. profitieren von dieser Sensorik.

Ein wichtiges Element der Pulsoximetrie ist, dass der Parameter Sauerstoffsättigung global ist, d.h. überall im Körper herrscht die gleiche arterielle Sauerstoffsättigung. Dies liegt daran, dass die Arterien bzw. Arteriolen das Blut lediglich verteilen, eine Diffusion von Sauerstoff findet aber nicht statt. Somit kann mittels der Pulsoximetrie die arterielle Sauerstoffsättigung an jedem Ort gemessen werden und die gemessene arterielle Sauerstoffsättigung ist für jeden anderen Ort des Körpers gültig.

Der Gehörgang als Messort für die Pulsoximetrie wurde nur wenig beachtet und auch nur unter recht speziellen Anwenungsszenarien wie Fighterpiloten und ähnliches - siehe US 05213099 und US 05662104.

US 05213099 schlägt einen elastischen Ohrstöpsel vor, der die optischen Komponenten für die Pulsoximetrie enthält. Die mechanische Anordnung ist hier simpel, Methoden zur Unterdrückung des Shuntlichts sind nicht beschrieben. Überlegungen zur Optimierung der Hilfsvariablen Omega oder zur Begrenzung von Bewegungsartefakten kommen ebenfalls nicht vor. Es wird von einem reflektiven Lichtweg durch das Gewebe gesprochen, was ein ungünstiges Lichtwegdesign darstellt, da die Reflex-Pulsoximetrie erhebliche Fehlerquellen beinhaltet. Die Druckschrift ist im 8. Jahr abgelaufen. Sie gibt ein Anfangsstadium der Ohr-Pulsoximetrie wider, das aber offensichtlich nicht weiter verfolgt wurde.

US 05662104 schlägt eine Klammer vor, bei der die eine optische Komponente im äußersten Bereich des Gehörgangs zu liegen kommt, die andere ist vollständig außerhalb. Die Klammer mit den beiden optischen Komponenten ist weit geöffnet und verspricht wenig Halt. Shuntlicht scheint ausgeschlossen. Der optische Pfad verläuft zwischen außen und innen, d.h. am äußersten Unterrand des Gehörgangeingangs und nicht im Gehörgang selbst. Das durchstrahlte Gewebe ist ein extrem flacher (stumpfwinkliger) Keil - kleinste Veränderungen der Klammer-Position, wie sie bei Bewegung unvermeidlich sind, verursachen schwerste Änderungen der Gewebsschichtdicke und damit ein nicht interpretationsfähiges Signal.

Beide Schriften zusammen zeigen recht deutlich, wie anspruchsvoll es ist, auch nur die wichtigsten Anforderungen der Pulsoximetrie gleichzeitig zu erfüllen. So löst die Anmeldung US 05662104 vorbildlich die Shuntlicht-Problematik, hat aber Probleme mit dem Halt des Sensors und mit bewegungsanfälligen Lichtwegen. Dagegen hat die US 05662104 stabile Verhältnisse der Gewebsschichtdicke, beachtet aber die Fehlerquelle Shuntlicht nicht. Dazu kommt, dass der Sensor mechanisch instabil ist, die Befestigung basiert ausschließlich auf der Expansion des Materials. Es fehlt die mechanische Beziehung zur Ohrmuschel.

***Messung mechanischer Größen wie Lage, Beschleunigung und Position:***

Mechanische Sensoren sind seit langer Zeit bekannt, allerdings sind erst in jüngster Zeit hinreichend kleine Sensoren verfügbar, die den Einzug in eine unauffällige mobile Sensorik ermöglichen z.B. ADXL330 4x4x1.4mm. Bisher nicht verfügbar sind mechanische Größen als mobile Sensorik und erst recht nicht in Kombination mit mobiler Sensorik anderer Parameter, die Teil der hier beschriebenen Erfindung sind.

## IV. Die Aufgabe der Erfindung

Die Überwachung mobiler Personen, d.h. unter Alltagsbedingungen setzt voraus, dass Sensoren verfügbar sind, die drei wichtigen Anforderungen genügen: Sie sollten erstens unter der Bedingung körperlicher Mobilität messen können, d.h. an einem Menschen, der einen normalen, unspektakulären Alltag absolviert, z.B. Arbeit oder Freizeit nachgeht. Sie sollten zweitens kosmetisch unauffällig zu sein, also im Alltag nicht irritieren, weder körperlich noch sozial, weder den Träger selbst noch seine Umgebung. Drittens sollten die Sensoren kontinuierlich, wenigstens häufig messen können, zumindest wiederholt. Dabei bedeutet "kontinuierlich", dass die Häufigkeit von Messungen so hoch ist, dass Änderungen der Messgröße mit einer Frequenz abgetastet werden (Gewinnung von Samples), die für alle diagnostischen und/oder therapeutischen Zwecke ausreicht, dass also eine quasi-kontinuierliche Aussage möglich ist. Entscheidend ist, dass die Problematik einer zu seltenen Messrate überwunden wird, ein Messfehler, der in der Medizin nicht selten vorkommt, insbesondere wenn Signalverläufe über längere Zeit z.B. über einen Tagesverlauf betrachtet werden sollen.

Sensoren, die diesen Forderungen genügen, sind sehr anspruchsvoll und innovativ im Vergleich zu Sensoren für einzelne Messwerte.

Im Einzelnen sind folgende Forderungen an eine moderne, mobile, im Alltag einsetzbare, kontinuierliche Sensorik zu stellen:

- die Sensoren sollten mobil sein, d.h. im Bezug auf die Wahl des Messortes und des Messprinzips so konzipiert sein, dass sie kontinuierlich Daten abgeben und dabei möglichst resistent sind gegenüber Störungen, die sich aus der Mobilität des zu Überwachenden ergeben
- mobile Sensoren sollten kosmetisch akzeptabel sein, d.h. sie sollten möglichst klein sein und wenig auffallen und den Alltag möglichst wenig behindern.
- mobile Sensoren sollten im Stromverbrauch so bemessen sein, dass mit den verfügbaren Energiezellen akzeptable Betriebszeiten verfügbar werden.
- mobile Sensoren sollten an geeigneten Messorten angebracht sein. So sind z.B. Messorte wie Mund, Speiseröhre, Rektum oder Vagina nicht zumutbar, Messorte wie Finger, Nase Stirn usw. nicht praktikabel.
- mobile Sensoren sollten am Körper so angebracht sein, dass sie schon primär möglichst wenig Störungen unter Normalbedingungen, d.h. während des bestimmungsgemäßen Einsatzes, z.B. im Alltag aufnehmen, seien es Störungen aufgrund von Bewegungen (Bewegungsartefakte) wie Stoß und andere mechanische Irritationen, seien es Störungen aufgrund von äußeren Einflüssen wie Sonnenbestrahlung, Irritationen durch Wasser, thermische, optische oder elektromagnetische Einflüsse.

**V. Beschreibung der Erfindung**

Die Erfindung bezieht sich auf Verfahren und Vorrichtungen zur Messung mindestens eines physiologischen und/oder biochemischen Parameters im Gehörgang eines Menschen oder eines Tieres, wobei wenigstens ein Sensorträger im Gehörgang angeordnet ist, wenigstens eine Sensorkomponente am Sensorträger angeordnet und/oder mit ihm verbunden ist, zur Messung mindestens eines physiologischen und/oder biochemischen und/oder bioelektrischen Parameters, wobei weiterhin ein Sensorträgerpositionierungselement, das die Vorrichtung im Gehörgang positioniert und die Eindringtiefe des Sensorträgers in den Gehörgang bestimmt, an einem Ende mit dem Sensorträger verbunden ist und vom Sensorträger im Gehörgang gehalten wird.

Erfindungsgemäß wird für die beschriebene innovative Sensorik der äußere Gehörgang als Messort herangezogen, der sich durch die Kombination von physiologischen Eigenschaften einerseits mit erfindungsgemäßen mechanischen, technischen und funktionellen Merkmalen andererseits gerade für die mobile bzw. die kontinuierliche Sensorik besonders gut eignet.

**1. Definitionen**

Für die Zwecke der vorliegenden Anmeldung werden folgende Definitionen zugrunde gelegt:

Unter einer *"kontinuierlichen Erfassung eines Parameters"* wird verstanden, dass entweder analoge Messwerte wirklich kontinuierlich zur Verfügung stehen oder dass diskrete analoge oder digitale Messwerte diskontinuierlich gewonnen werden können, in einer zeitlichen Dichte, die hoch ist, bezogen auf die Kinetik des Parameters und die damit ausreichend ist für den angestrebten diagnostischen oder therapeutischen Zweck. Die notwendige Messwertdichte hängt also von der zeitlichen Kinetik ab, mit der sich ein Parameter verändern kann.

Unter "*Sensordesign*" wird primär der Aufbau des Sensors hinsichtlich seiner Funktionalität insbesondere hinsichtlich der Signalqualität verstanden - eine Formgebung unter ästhetischen Gesichtspunkten ist ein nachgeordneter Gesichtspunkt.

Unter "*Licht*" wird elektromagnetische Strahlung im Bereich von 400 nm bis 2500 nm, insbesondere im Bereich zwischen 600 nm und 2000 nm, verstanden.

Unter "*Sensorkomponente*" wird der Sensor im engsten Sinn verstanden, also die Vorrichtung, die die eigentliche physiologische bzw. biologische Größe in ein elektrisches Signal umwandelt, beispielsweise Wandler, Halbleiter, Messfühler, Elektroden.

Unter *"Sensorträger"* und *"Sensorträgerpositionierungselement"* [in früheren Anmeldungen auch als Sensorträgerformschlauch bezeichnet] werden funktionell und mechanisch wirkverbundene Vorrichtungen verstanden, die zusammen die Sensorkomponente relativ zur Gehörgangswand so positionieren, dass der physiologische bzw. biologische Parameter möglichst störungsarm gemessen werden kann. Unter formal logischen Gesichtspunkten ist es die Aufgabe des Sensorträgers die radiale Position der Sensorkomponente zu definieren, während es die Aufgabe des Sensorträgerpositionierungselements ist, die axiale Position zu definieren. In konkreten Ausführungsformen sind diese beiden Funktionen oft nicht trennbar bzw. sie verschmelzen miteinander.

Unter "*Sensor bzw. Sensorvorrichtung*" wird die Kombination aus Sensorkomponente, Sensorträger, Sensorträgerpositionierungselement, Mini-Auswerteeinheit und anderen Vorrichtungen verstanden, die zusammen den Sensor bilden. Erst das Zusammenwirken einiger oder aller dieser Komponenten mit wichtigen Prinzipien des Sensordesigns und anderer erfindungsgemäßer Methoden ergeben die angestrebte Funktionalität des Sensors.

Unter *"Absorption"* werden hier zwei eigentlich unterschiedliche optische Phänomene subsumiert: einerseits wird darunter die Lichtintensität verstanden, die nach der Passage durch das Gewebe am Lichtempfänger messbar ist, unabhängig davon, ob die eingestrahlte Lichtintensität oder die elektrische Leistung bekannt ist; andererseits wird darunter verstanden das Verhältnis der Lichtintensität nach der Passage durch das Gewebe bzw. seine Substrukturen zur ins Gewebe bzw. seine Substrukturen eingestrahlten Lichtintensität.

Unter "*Shuntlicht*" werden zwei Phänomene der Lichtausbreitung in Gewebe, insbesondere der Pulsoximetrie verstanden:

"*Shuntlicht*" bedeutet, dass Licht von Lichtemitter zum Lichtempfänger auf direktem Weg zum Lichtempfänger gelangt. "*Shuntlicht*" im engeren Sinn bedeutet, dass Licht von Lichtemitter zum Lichtempfänger auf direktem Weg zum Lichtempfänger gelangt, ohne auf seinem Weg *durchblutetes Gewebe* durchstrahlt zu haben. Wird also Gewebe durchstrahlt, das nicht durchblutet ist, in dem insbesondere keine Arteriolen verlaufen, so handelt es sich um Shuntlicht im engeren Sinn. Bei der Reflex-Pulsoximetrie lässt sich Shuntlicht im engeren Sinn praktisch nicht vermeiden, weil das Licht auch Gewebe durchstrahlt, das nicht durchblutet ist, z.B. Hornhaut

bzw. äußere Hautschichten. Wird das Licht an der Geweboberfläche, z.B. der Hautoberfläche, erreicht ein Teil des Lichts den Empfänger. Dieses Shuntlicht hat das Gewebe nicht durchstrahlt.

Bei der Transmissions-Pulsoximetrie kommt praktisch kein Shuntlicht im engeren Sinn vor, weil kein Licht den Empfänger erreicht, das nicht-durchblutetes Gewebe durchstrahlt hat.

Shuntlicht muss bei der Konstruktion eines optischen Sensors, d.h. beim Sensordesign bestmöglich ausgeschlossen werden.

Unter *"Netto-Lichtweg"* wird der kürzeste Lichtweg verstanden, den das Licht vom Austritt aus dem Lichtemitter bis zum Eintritt in den Lichtempfänger benutzen kann - unter Einbeziehung von Streu- und Beugungsphänomenen im vitalen Gewebe. Auf dem Weg vom Lichtemitter zum Lichtempfänger sind beliebig viele Ausbreitungswege unterschiedlicher Länge denkbar, möglich bzw. realisiert, abhängig von den multiplen Stellen / Streuzentren, an denen Lichtstrahlen auf diesem Weg gebeugt, gebrochen und gestreut werden können. Der "Netto-Lichtweg" repräsentiert aber den besonderen Lichtweg mit der höchsten Lichtintensität am Lichtempfänger - unter Einbeziehung von Streu- und Beugungsphänomenen im vitalen Gewebe.

Unter *"mechanische Parameter"* werden Größen wie Lage, Position, Beschleunigung und davon (durch Integration bzw. Differenzierung) abgeleitete Größen subsumiert.

## 2. Die erfindungsgemäßen Grundkomponenten

Die jeder Sensorik zugrunde liegende Sensorkomponente wird so mit dem Gewebe in eine mechanische und funktionelle Beziehung gebracht, dass ein initiales Signal gewonnen werden kann, aus dem sich der Parameter möglichst störungsarm aufbereiten lässt. Erfindungsgemäß wurde diese Forderung so umgesetzt, dass die eigentliche Sensorkomponente einerseits eine *mechanische Beziehung* zum Gewebe hat, gegebenenfalls eine stabile Positionierung in Form einer Berührung oder eines definierten Andrucks, andererseits eine *funktionelle Beziehung,* die in einer Beeinflussung der Sensorkomponente(n) durch den bzw. die zu messenden physiologischen Parameter bzw. durch dessen Änderung z.B. Temperatur oder Druck aber auch in der Beeinflussung einer physikalischen Größe durch das Gewebe des Gehörgangs bzw. durch dessen anatomische bzw. physiologische Bestandteile z.B. die Absorption von Licht im Gehörgang insbesondere die Änderung der Absorption durch die im Gehörgang befindlichen Gefäße oder durch biochemische Substanzen besteht. Natürlich werden Sensorkomponenten auch durch Störgrößen beeinflusst, bei mobilen Anwendungen sind dies insbesondere Bewegungsartefakte, aber auch Feuchtigkeit, Temperaturänderungen usw.. Dies wird vorzugsweise beseitigt bzw. wenigstens berücksichtigt.

Ein weiterer Aspekt der Erfindung betrifft insbesondere die Kombination folgender Elemente:

✦ die Wahl des äußeren Gehörgangs als Messort ist in vielfacher Hinsicht günstig: erstens sind Trage-Komfort und Trage-Kosmetik im äußeren Gehörgang sehr günstig; zweitens sind Bewegungen des Kopfes im Vergleich zu Extremitäten eher gering und niederfrequent, auch werden heftige Kopfbewegungen meist als unangenehm empfunden und daher vermieden (Mikrobewegungen, wie sie beim Reden oder Kauen auftreten, müssen beim Tragen des Sensors, sowie bei dessen Design und schließlich bei der Signalauswertung berücksichtigt werden); drittens ist der äußere Gehörgang gut durchblutet, was für optisch-plethysmografische Messprinzipien wie die Pulsoximetrie eine wichtige Voraussetzung ist; viertens ist der äußere Gehörgang nicht in die Atmung einbezogen; fünftens ist der äußere Gehörgang ein Bereich trockener Haut;

✦ die günstige Positionierung der Sensorkomponente mittels der Gesamt-Vorrichtung umfasst: Sensorkomponente, Sensorträger, Sensorträgerpositionierungselement sowie Mini-Auswerteeinheit hinter der Ohrmuschel, von der jede einzelne Komponente mit zur Positionierung bzw. zum Halt beiträgt - erstens relativ zur Wand des Gehörgangs (mittig oder exzentrisch oder wandständig) in Abhängigkeit des zu messenden Parameters und zweitens hinsichtlich ihrer Eindringtiefe;

★ der Signalverlauf umfasst die primäre Sensorik zusammen mit einer rauscharmen Analog-Signalverarbeitung beispielsweise in der Mini-Auswerteeinheit hinter dem Ohr, gekoppelt evtl. mit Frühdigitalisierung der analogen Signale und dem Versenden digitaler Informationen an weitere Peripherie wie Mobilfunkeinheit, Computer usw., siehe unten.

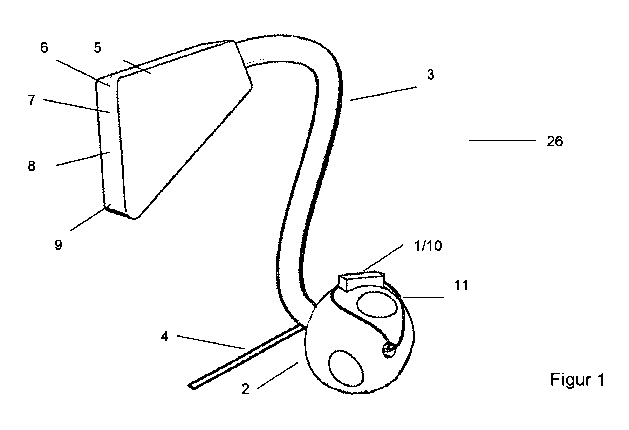

Ein weiteres, allen Ausführungsformen gemeinsames Charakteristikum ist, dass der Schall weitgehend ungehindert durch die verschiedenen Komponenten der Sensorik ans Trommelfell gelangt. Dies wird entweder erreicht, indem die verschiedenen Komponenten der Sensorik den Schall eben nicht oder nicht nennenswert dämpfen, dass insbesondere der Sensorträger so konstruiert ist, dass er die Schallfortleitung in Richtung auf das Trommelfell entweder zulässt, d.h. nicht wahrnehmbar behindert, oder sie sogar aktiv herstellt. Dazu darf der Sensorträger den äußeren Gehörgang nicht vollständig verschließen oder bedämpfen - es sind geeignete Löcher bzw. Aussparungen bzw. Kanäle vorzusehen, die dies gewährleisten (siehe z.B. Figuren 1, 2, 8, 10). Es ist auch möglich, dass eine aktive Schallverbindung zwischen dem äußeren Ohr und dem Trommelfell hergestellt wird, falls aufgrund der Messaufgabe eine Schalldämpfung durch die verschiedenen Komponenten der Sensorik unvermeidbar ist. Die aktive Schallverbindung besteht unter anderem aus einem kleinen Mikrofon im Bereich der Ohrmuschel und einem winzigen Lautsprecher im hinteren Gehörgang, in Richtung auf das Trommelfell.

Die im Folgenden beschriebenen Ausführungsformen sind beispielhaft und unterscheiden sich hinsichtlich des zu messenden physiologischen Parameters und hinsichtlich des für die jeweilige Messaufgabe notwendigen spezifischen Sensordesigns.

Der *Sensorträger* ist die Vorrichtung, die die Sensorkomponente im Gehörgang positioniert. In vielen Ausführungsformen ist die Nähe der Sensorkomponente zur Wand des Gehörgangs eine wichtige funktionelle Größe des Sensordesigns, die eben durch den Sensorträger gewährleistet wird. Dazu weist der Sensorträger die Eigenschaft auf, sich dehnen bzw. spreizen zu können. Daher haben viele Ausführungsformen elastische bzw. expansive Eigenschaften bzw. ein Formgedächtnis, häufig ist der Sensorträger z.B. aus Kunststoff, gegebenenfalls aus Silikon hergestellt und/ oder aus einem dünnen, federnden Blech. Der Sensorträger kann z.B. die Form einer runden oder elliptischen Scheibe haben oder eines kleinen Sternchens beispielsweise mit zwei, drei oder mehreren Berührungspunkten der Gehörgangswand oder auch beliebig vielen, d.h. einer Berührungslinie bzw. Berührungsflächen oder die Form einer U- oder S-förmigen Spange mit zwei oder mehr Berührungspunkten bzw. Berührungsflächen.

Eine besonders vorteilhafte Ausführungsform des Sensorträgers umfasst eine Schirmchen-Form, d.h. der Sensorträger mit seinen elastischen bzw. federnden Eigenschaften breitet sich einerseits senkrecht zur Gehörgangsachse aus bis er den Gehörgangs-Querschnitt kreis- bzw. ellipsenförmig erfüllt, andererseits wölbt sich der Sensorträger über die gesamten Umfang zum Rand hin in Richtung der Gehörgangsachse, wodurch eine mehr oder weniger breite Berührungslinie entsteht, in deren Bereich Sensorkomponenten ideal positioniert werden können: der Andruck ist formbedingt zuverlässig und stabil und kaum abhängig vom Federweg, gleichzeitig sanft und sicher.

Durch die beschriebene Form und durch die genannten Materialeigenschaften zentriert sich der Sensorträger im Gehörgang, wobei er den Gehörgang entweder rundum berührt oder nur an einigen wenigen Punkten, beispielsweise 2, 3 oder 4, mit einer Auflagefläche, die mit der Federkraft des Sensorträger derart in Beziehung steht, dass der Andruck den der Sensorträger auf die Wand des Gehörgangs, einen Druck von unter 40mm Hg nicht übersteigt, damit keine Durchblutungsstörungen im Gewebe auftreten und verbunden damit auch keine (Hypoxie-) Schmerzen beim längerfristigen Tragen des Sensors und erst recht keine Gewebsschädigung. Da der Gehörgang oft eher oval als kreisrund ist, eignen sich auch nicht-kreisförmige Sensorträger gut. Wichtig ist, dass die Form und die Materialeigenschaften des Sensorträgers eine Zentrierung beim Einsetzen des Sensorträgers im Gehörgang erlauben, ja begünstigen.

Das *Sensorträgerpositionierungselement* ist die Vorrichtung, die die axiale Position der Sensorkomponente im Gehörgang definiert, d.h. ihre Tiefe im Gehörgang, eine Größe, die für das Messergebnis wichtig ist.

Das Sensorträgerpositionierungselement ist auch wesentlich für den Halt, d.h. für die mechanische Stabilität der gesamten Sensorvorrichtung zuständig. Dieser Halt und die mechanische Stabilität werden durch mehrere Merkmale des Sensorträgerpositionierungselements erreicht:

- durch eine enge, vorwiegend radial wirksame mechanische Beziehung mit dem Gehörgang über den Sensorträger,
- über eine enge mechanische Beziehung zur Innenseite der Ohrmuschel über einen Haltefaden und über eine geeignete, die Anatomie der Ohrmuschelinnenseite nachvollziehende Formgebung des Sensorträgerpositionierungselements
- über eine enge mechanische Beziehung zur Mini-Auswerteeinheit hinter dem Ohr, die ihrerseits Halt findet durch Einklemmung gegen den Kopf
- durch die Materialeigenschaften des Sensorträgerpositionierungselements, die durch ein Formgedächtnis des Materials bewirken, dass jede Teilkomponente der mechanischen Beziehungen sich mit den anderen Teilkomponenten zu einem guten Sitz der Sensorvorrichtung insgesamt verbindet und dadurch zu einem geringen Ausmaß an Bewegungsartefakten.

Das Sensorträgerpositionierungselement wird also vorzugsweise aus einem gewebsverträglichen Material hergestellt, das ein Formgedächtnis besitzt z.B. kann man Kunststoffe wie Polypropylen oder Polyethylen oder Metalle dafür einsetzen. Es ist typischerweise ca. 6 bis 7 cm lang und hat einen Durchmesser zwischen 0.5 mm und 3 mm.

Form und Länge des Sensorträgerpositionierungselements und schließlich auch die Länge des Haltefadens werden vorzugsweise den individuellen Gegebenheiten einerseits und der Trageseite (rechtes Ohr versus linkes Ohr) andererseits angepasst, denn Formgebung des Sensorträgerpositionierungselements, sowie der Abgang des Kunststoffpositionierungsfadens für rechtes und linkes Ohr sind zueinander spiegelbildlich.

Alle Komponenten, auch die Drähte und Lichtleiter werden vorzugsweise hautfarben eingefärbt, so wird ein solcher Art aufgebauter Sensor besonders unauffällig.

Das Sensorträgerpositionierungselement kann man in drei wesentliche Teilbereiche gliedern, die sich durch zwei Biegungen dazwischen ergeben: Die erste Biegung von ungefähr 90° des Sensorträgerpositionierungselements trennt den Teil des Sensorträgerpositionierungselements der im Gehörgang liegt von dem Teil, der durch den konvexen Innenhelix der Ohrmuschel nach oben (kranial) verläuft. Die zweite Biegung von typischerweise mehr als 90° verläuft um die obere Umschlagfalte der Ohrmuschel nach hinten zur Mini-Auswerteeinheit hinter der Ohrmuschel. Diese Mini-Auswerteeinheit ist in ihrer Form vorzugsweise so an diesen Raum adaptiert, dass sie einen möglichst großen Raum für Energieversorgung und Elektronik zur Verfügung stellt, aber auch gut Platz findet und guten Halt findet zwischen der Ohrmuschel und Kopfhaut und die sich dort stabil einklemmt.

Der Haltefaden geht mehr oder weniger rechtwinklig vom Sensorträgerpositionierungselement ab und ist flexibel, um sich in die hintere Krümmung der konvexen Helix der Ohrmuschel einzufügen. So wird dem Sensorträgerpositionierungselement und damit der gesamten Sensorvorrichtung zusätzlicher Halt gegeben. Der Haltefaden besteht aus einem runden, flexiblen, Material, es kann ein Kunststoff-Faden also Vollmaterial oder ein Kunststoffschlauch von beispielsweise 1 bis 2.5 mm Durchmesser verwendet werden.

Dazu hat das Sensorträgerpositionierungselement mit dem Sensorträger eine formschlüssige, trennbare Verbindung z.B. eine Schnapp- bzw. Rastverbindung d.h. eine kleine Verdickung im Sensorträgerpositionierungselement und eine kleine verstärkte (Mittel-) Öffnung im Sensorträger sind so aneinander angepasst, dass sie ineinander "einrasten" können. Diese optimale Gehörgangstiefe hängt von den anatomischen Gegebenheiten ab, so dass für diese Länge des Sensorträgerpositionierungselements einige typische Längen vorgehalten werden müssen. Der Sensorträger mit dem/den Sensor(en) wird dann mit dem bestpassenden Sensorträgerpositionierungselement formschlüssig verbunden.

Das Sensorträgerpositionierungselement ist meist schlauch- oder röhrchenförmig, d.h. innen hohl, und stellt so eine mechanische und/oder optische und/oder elektrische Verbindung und/oder druck-leitende Verbindung (Luft / Wasser u.a.) her zwischen der Sensorkomponente einerseits und der Mini-Auswerteeinheit hinter dem Ohr andererseits, wobei zum Beispiel die Lichtleiter oder die elektrischen Drähte im Inneren des Sensorträgerpositionierungselements zu liegen kommen und so Signale und Potentiale übertragen.

Das Sensorträgerpositionierungselement kann aber auch als Leitstruktur dienen, beispielsweise für die Anschlussdrähte der Sensorkomponente bzw. der Licht-Gas- oder Flüssigkeitsleiter. Beispielsweise können die Anschlussdrähte im Kunststoff bzw. zusammen mit ihm extrudiert werden oder auf andere Weise mit dem Sensorträgerpositionierungselement verbunden sein. Werden Drähte durch das Sensorträgerpositionierungselement durchgeführt, so können sie z.B. im Gehörgang, nahe dem Schirmchen in das Sensorträgerpositionierungselement eintreten und treten dann in der Mini-Auswerteeinheit hinter der Ohrmuschel wieder aus dem Sensorträgerpositionierungselement aus.

Es kann zwischen Sensorträger und Sensorträgerpositionierungselement auch fließende Übergänge geben, d.h. es gibt Ausführungsformen, bei denen keine klare Abgrenzung zwischen diesen beiden Komponenten zu erkennen ist. Es ist dann diejenige Komponente, die sich in den Gehörgang erstreckt als Sensorträgerpositionierungselement anzusehen, diejenige, die die Sensorkomponente trägt als Sensorträger, unabhängig davon, ob eine äußere Abgrenzung möglich ist. Es gilt dann eine funktionelle Unterscheidung.

Die **Mini-Auswerteeinheit** hinter dem Ohr am von der Sensorkomponente abgewandten Ende des Sensorträgerpositionierungselements ist durch ihren guten Halt infolge ihrer "Einklemmung" ein Element der mechanischen Fixierung der gesamten Sensorvorrichtung. In einer vorteilhaften Ausführungsform enthält sie die Energiezellen sowie gegebenenfalls einen Teil der Elektronik für die Signalverarbeitung. Sie enthält gegebenenfalls auch Elektronik für die Signalweiterleitung z.B. via infrarot oder per Funk z.B. via Bluetooth oder Zigbee oder eine andere Realisierung einer drahtlosen, digitalen oder analogen Übertragungsfunktion. Als Folge der Platzbeschränkung kann die Mini-Auswerteeinheit hinter dem Ohr auch nur eine Telemetrieeinheit sein, d.h. die von ihr geschickten Signale werden an die eigentliche Auswerte- und/oder Speichereinheit weitergereicht und dort noch weiter prozessiert bevor sie vom Benutzer genutzt werden können. Die Mini-Auswerteeinheit hinter dem Ohr muss nicht zwingend die beschriebenen Komponenten enthalten, sie kann völlig leer sein und ausschließlich als funktionelle Komponente für den Halt der Sensorvorrichtung dienen. Für die erfindungsgemäße Funktionalität ist es unwichtig, ob die jeweiligen Module Mini-Auswerteeinheit hinter dem Ohr sowie die Anzeigeeinheit bzw. die Auswerteeinheit und die Mobilfunkeinheit tatsächlich räumlich getrennte Einheiten sind. Je nach erwünschter Funktionalität und je nach erreichbarer Miniaturisierung können diese Module beliebig zusammengefasst werden oder auch ganz entfallen.

Im Folgenden werden Ausführungsformen unter funktionellen Gesichtspunkten beispielhaft ausgeführt. Die Ausführungsformen sind für bestimmte Messaufgaben bzw. bestimmte Parameter speziell konzipiert.

**Parameter Körper(kern)temperatur:**

Zur Messung der Körper(kern)temperatur sollte auf folgende Merkmale der Ausführungsform(en) geachtet werden: ein oder mehrere winzige auf Konvektion basierende Temperatursensoren d.h. Berührungssensoren werden so auf dem Sensorträger angeordnet, dass sie vorzugsweise den mittleren oder hinteren Gehörgang thermisch schlüssig berühren. Platinwiderstandsfühler, z.B. Pt100, PT500 oder Pt1000 Widerstandstemperatursensoren eignen sich besonders gut wegen ihrer weitgehend linearen und relativ steilen Kennlinie aber auch NTCs eignen sich.

Eine vorteilhafte Ausführungsform weist eine Anordnung auf, bei der mehrere Temperatursensoren auf dem Sensorträger positioniert sind, um mehrere Temperaturinformationen zu erhalten. Da die Temperatur immer eine räumlich und zeitlich verteilte Größe darstellt, gilt es dann eine geeignete zu wählen. Das könnte beispielsweise die maximale Temperatur sein, da sie gut mit der Körper(kern)temperatur korreliert. Aus der Temperaturverteilung an den einzelnen Sensoren kann aber auch auf die Qualität der Messsituation zurück geschlossen werden, u.a. auf die Qualität des thermischen Kontakts.

Prinzipiell kann man auch einen Strahlungssensor einsetzen, der auf dem Sensorträger positioniert wird und dessen Einfallswinkel typisch in Richtung auf den inneren Teil des Gehörgangs gerichtet ist, d.h. das Trommelfell oder den Gehörgang in der Nähe des Gehörgangs.

Eine weitere Ausführungsform besteht darin, eine dünne Platinschicht als Temperatursensor einzusetzen, die beispielsweise mäanderförmig auf den Rand des Schirmchens aufgebracht wurde. Diese mäanderförmige Widerstandsschicht kann den gesamten kreisförmigen Berührungsstreifen einnehmen, aber auch nur Teile davon.

Wenn als Messprinzip ein Konvektionssensor gewählt wird, muss der Temperatursensor in jeder Form, sowohl als kompakter, miniaturisierter Sensor jedweder Technologie, als auch als Schichtsensor an das Gewebe mit einem Druck angedrückt werden, der so gering ist, dass er keine Irritationen am Gewebe hervorruft aber doch groß genug, um einen zuverlässigen thermischen Kontakt zum Gewebe herstellen zu können.

Für die Messkinetik ist es günstig, wenn der Temperaturfühler selbst eine kleine thermische Masse besitzt und wenn sich diese thermische Masse durch die angrenzenden Materialien, z.B. durch den Sensorträger, nicht nennenswert vergrößert. Dies kann erreicht werden, indem der Temperaturfühler entweder thermisch möglichst isoliert ist vom Material des Sensorträgers oder wenn der Sensorträger selbst eine geringe Wärmekapazität und/oder Wärmeleitung besitzt. Wichtig ist auch ein guter thermischer Kontakt zur Wand des Gehörgangs. Durch die beschriebenen Maßnahmen lässt sich erreichen, dass der Einfluss des Körpers groß, der Einfluss der Umgebung dagegen klein bleibt.

Eine häufige Fehlerquelle ist der Austausch der Luft im Gehörgang durch Umgebungsluft. Erfindungsgemäß kann dieser Störquelle in mehrfacher Weise entgegengewirkt werden: Erstens wird dieses Problem desto geringer und die gemessene Temperatur entspricht desto genauer der Körper(kern)temperatur je weiter innen im Gehörgang der Sensor angeordnet wird, je weniger also die Temperatur durch Luft von außen kontaminiert wird. Zweitens lässt sich der Luftaustausch mit Umgebungsluft reduzieren z.B. indem man den äußeren Gehörgang etwas abdichtet beispielsweise mittels eines porösen Stöpsels bzw. indem man die Ohrmuschel mehr oder weniger locker bedeckt beispielsweise mit einem Ohrschutz, Kopftuch, Stirnband oder einer Mütze. Drittens kann man die Störgröße bestimmen: dazu misst man den Temperaturgradienten zur Außenwelt durch mehrere Sensorkomponenten und zieht diese Information zur Korrektur der Störgröße durch die äußeren Einflüsse heran. Viertens kann man die Störgröße reduzieren: dazu wird im Außenbereich des Gehörgangs eine Vorrichtung angebracht, die den Gradienten zwischen der Körper(kern)temperatur und der Temperatur in der Außenwelt reduziert, z.B. eine Art Heizung oder Kühlung, je nach Richtung des Gradienten. Mit der Reduzierung des Gradienten sinkt der Messfehler.

Die Körper(kern)temperatur im Ohr zu messen gelingt desto genauer, je tiefer die Sensorkomponente im äußeren Gehörgang sitzt. Dem sind aber Grenzen gesetzt, da jede Berührung im äußeren Gehörgang desto unangenehmer wird, je mehr man sich dem Trommelfell nähert.

Die Verwendung des äußeren Gehörgangs zur Messung der Körper(kern)-temperatur setzt den Andruck des Berührungssensors gegen den Gehörgang voraus. Sollte dieser z.B. wegen fehlerhaftem Einsetzen des Sensors nicht gegeben sein, so kann dies erfindungsgemäß erkannt und berücksichtigt werden und zwar vorteilhafterweise über die Wärmekapazität und/oder Wärmeleitung der Materie, die die Sensorkomponente umgibt: Luft hat eine geringe Wärmeleitfähigkeit bzw. Wärmekapazität, Gewebe hat eine große Wärmeleitfähigkeit bzw. Wärmekapazität. Für die Unterscheidung der umgebenden Materie wird die Sensorkomponente auf Übertemperatur gebracht, d.h. es wird in die Messung der Körpertemperatur eine Heizphase eingelegt, in der die umgebende Materie auf eine Übertemperatur gebracht wird. Unmittelbar nach der Heizphase (Messphase) wird die Temperatur bzw. der Temperaturabfall gemessen. Aus der Kinetik des Wärmeverlustes kann man auf die umgebende Materie geschlossen werden, denn Luft kühlt viel schneller ab als Gewebe. Elegant ist die Verwendung nur eines Messwiderstands für alle Heiz- und Messaufgaben.

*Parameter Blutdruck:*

Erfindungsgemäß wird der äußere Gehörgang als Messort für die Blutdruckmessung herangezogen. Erfindungsgemäß werden dazu entweder mechanische oder optische Möglichkeiten herangezogen. Dabei ist das Sensordesign das eigentliche erfinderische Merkmal, das Verfahren zur Messung des Blutdrucks kann dabei dem Stand der Technik entsprechen. Da aufgrund der geringen Gewebsschichtdicke der Gehörgangswand auch nur geringe plethysmografische Volumenverschiebungen auftreten, d.h. kleine pulsierende Blutvolumina $\Delta V$ vorliegen, sind Effekte, die sich auf Volumenverschiebungen beziehen sehr klein. Es gilt daher, druckbasierte Messtechniken vorzuziehen, da auch im Gehörgang prinzipiell gleiche Blutdruckverhältnisse vorliegen

Bei einer Ausführungsform wird das Gewebe des äußeren Gehörgangs zentrifugal komprimiert. Im Gegensatz zu Blutdruckmanschetten an Extremitäten, die sich zur zentralen Achse hin komprimieren, findet hier die Kompression von der zentralen Achse weg nach außen hin statt, mittels einer expansiven Manschette, d.h. einem walzenförmigen Ballon, der gegen den äußeren Gehörgang durch Auffüllen mit einem Gas(-gemisch), vorteilhafter mit einer Flüssigkeit expandiert wird. Die Blutdruckmanschette im äußeren Gehörgang arbeitet also expansiv. Das Volumen der expansiven Manschette sollte gering sein, damit deren Kompression messbar bleibt und zweitens sind inkompressible Medien zur Weiterleitung der intraarteriellen Druckänderungen vorzuziehen und drittens sollten alle Teile der expansiven Manschette, die nicht mit pulsierenden Teilen des Körpers in Berührung sind, möglichst starr sein, damit möglichst wenig des plethysmografisch verschobenen Blutvolumens $\Delta V$ durch irgendwelche Verformungen von Schlauchwänden oder Verformungen von Manschettenwänden oder durch Kompression von Gasen verbraucht wird bzw. verloren geht.

Dazu sollte die expansive Manschette natürlich nur in dem Bereich verformbar sein, in dem sie den Gehörgang berührt. Zur Messung des Drucks in der expansiven Manschette ist entweder eine Druckmessvorrichtung bereits innerhalb der expansiven Manschette angeordnet oder der Druck wird an eine Druckmessvorrichtung weitergeleitet, beispielsweise durch das Sensorträgerpositionierungselement, der zu diesem Zweck natürlich relativ druckstabil sein sollte. Das Sensorträgerpositionierungselement dient als Vorrichtung zur korrekten Positionierung der Sensorkomponente, beispielsweise der expansiven Manschette und gegebenenfalls einer Druckmessvorrichtung in ihrem Inneren oder unmittelbar daneben und/oder der Fortleitung der Signale z.B. elektrischer Signale einer Druckmessvorrichtung, gegebenenfalls aber auch, um eine gas- oder flüssigkeitsführende Verbindung zur Pumpe und/oder zum Druckmessglied herzustellen, die beispielsweise in der Mini-Auswerteeinheit hinter der Ohrmuschel angeordnet sind. Eine weitere vorteilhafte Ausführungsform einer solchen Blutdruckmessvorrichtung besteht in der Verwendung kleiner Detektorflächen, kleinen Druckmessvorrichtungen also, die im Gehörgang kleinflächig aufsetzen, die so einander gegenüber liegen oder die im 120°-Winkel angeordnet sind, dass sich die Sensoren selbst zentrieren, bzw. die Effekte gegenüberliegender Wände sich gegenseitig verstärken. Es ist vorteilhaft, wenn die Elemente, die den Druck messen können und die Elemente, die den Druck auf die Wand des Gehörgangs ausüben, um die arteriellen Gefäße bis zum Verschluss und noch darüber hinaus zu komprimieren.

In einer weiteren erfindungsgemäßen Ausführungsform wird der Ausschlag bzw. werden die Druckschwankungen in Abhängigkeit vom Druck auf die Wand des Gehörgangs, d.h. die oszillometrische Information optisch gewonnen.

Die Bestimmung des Blutdrucks wird vorzugsweise aus den beiden Teil-Informationen Druck auf das Gewebe des Gehörgangs einerseits und die relative, aus den Arterien und Arteriolen des Gewebes auf das Druckmesssystem übertragene, pulsfrequente Druckamplitude andererseits abgeleitet, d.h. sie erfolgt beispielsweise auf der Basis der oszillometrischen Messtechnik. Der systolische Druck kann auch als arterieller Verschlussdruck beispielsweise optisch gewonnen werden, z.B. mittels der Unterdrückung optisch-plethysmografischer Phänomene. Dazu können die notwendigen optischen Komponenten in die expansive Manschette integriert bzw. in seiner unmittelbaren Umgebung platziert werden.

Gegebenenfalls kann die expansive Manschette auch zum vorübergehenden Schutz des Ohres vor exzessivem Lärm eingesetzt werden.

Bei der Berechnung des im Gehörgang detektierten Blutdrucks ist der (negative) hydrostatische Druck zu berücksichtigen, der durch den Abstand vom äußeren Gehörgang zum Herzen zustande kommt. In diesem Zusammenhang ist es sinnvoll, einen Taster oder Schalter vorzusehen, mit dem der Benutzer dem Blutdruckmessgerät die Körperhaltung übergeben kann, entweder senkrecht (=stehend, sitzend) oder waagrecht (=liegend). Diese Information kann aber auch von der Mini-Auswerteeinheit selbst durch einen oder mehrere Inklinationssensor(en) festgestellt werden.

Ein Vorteil dieser Messtechnik ist, dass der Anwender nach einer einmaligen Kalibration, die den Abstand vom äußeren Gehörgang zum Herzen berücksichtigt, keine Fehlmessungen aufgrund veränderlichen hydrostatischen Drucks erhalten kann, wie es sonst bei unterschiedlichen Positionen des Messarms relativ zum Herzen der Fall ist.

Die Messung des Blutdrucks kann mit der erfindungsgemäßen Vorrichtung bzw. unter Anwendung der erfindungsgemäßen Methodik sowohl in Ruhe als auch unter mobilen Bedingungen erfolgen. Die Messung in Ruhe ermöglicht insbesondere ein Screening hinsichtlich der Diagnose einer (essentiellen) Hypertonie aber auch die Bewertung einer antihypertensiven Therapie. Die diagnostische Bedeutung des Parameters Blutdruck unter mobilen Bedingungen, insbesondere unter Belastung ist heute noch kaum abschätzbar. So könnte die Messung des Blutdrucks z.B. beim Sport eine Bewertung des Trainingserfolgs und bestimmter Überlastungs- bzw. Erschöpfungszustände ermöglichen. Auch spiegelt sich der Wasserhaushalt im Blutdruckprofil wider. Letztlich sind der Blutdruck und die Herzfrequenz, eventuell auch in Kombination mit der Körper-(kern)temperatur auch Komponenten einer Beurteilung der Vigilanz was besonders für Fahrer von KFZs von erheblicher Bedeutung ist.

***Parameter EKG:***

Die Erfassung von Potentialdifferenzen z.B. fortgeleitetes EKG, erfolgt erfindungsgemäß an unterschiedlichen Orten im bzw. am Ohr. Wenigstens eine Elektrode befindet sich im Gehörgang so auf dem Sensorträger, dass ein sicherer elektrischer Kontakt zur Wand des Gehörgangs besteht. Eine weitere Elektrode befindet sich z.B. hinter der Ohrmuschel, ist also beispielsweise der Mini-Auswerteeinheit hinter dem Ohr zugeordnet. Je weiter die zweite Elektrode von der ersten entfernt ist, desto größer ist die Potentialdifferenz und desto größer der S/N-Abstand. So könnte die zweite Elektrode auch contralateral am anderen Ohr positioniert werden, besser am Arm, beispielsweise im Sinne einer Armbanduhrähnlichen Elektrode, noch besser in (elektrischer) Nähe des Thorax. Prinzipiell können auch Potentialdifferenzen anderen Ursprungs erfasst werden. Als Elektrode im Gehörgang eignet sich eine auf einem schirmchenförmigen Sensorträger zirkulär verlaufende Schicht, die an vielen Stellen Berührung zum Gehörgang hat, ähnlich wie die meanderförmige Temperatur-Sensor-Schicht bei der Sensorkomponente zur Messung der Körpertemperatur, jedoch ohne den hohen Temperaturkoeffizienten.

***Messung fotometrischer und optisch-plethysmografischer Größen:***

Die Vorteile, z.B. für die Pulsoximetrie anstelle eines Fingerendglieds den äußeren Gehörgang als Messort zu verwenden sind vielfältig: so sind optische Sensoren im Gehörgang vergleichsweise gut lichtgeschützt, d.h. es ist im Vergleich zum Fingersensor mit weniger Fremdlicht zu rechnen. Auch treten am Kopf weniger Beschleunigungen auf als an einer Extremität, erst recht als am Finger, der eine besonders gute Beweglichkeit besitzt. Ein weiterer entscheidender Vorteil des Messortes "Gehörgang" ist aber, dass ein Gehörgangssensor deutlich weniger Irritation bzw. Behinderung bedeutet als ein Fingersensor. Vor nicht unerheblicher Bedeutung ist auch, dass ein gut konzipierter und hochgradig miniaturisierter Gehörgangssensor kaum auffällt, er wird kaum wahrgenommen. Möglicherweise lässt sich sogar ein Trend etablieren, d.h. ein Gehörgangssensor wird mit entsprechenden modischen dekorativen Merkmalen ausgestattet, sodass er als "trendy" aufgefasst und so sogar gerne getragen wird.

Verschiedene zu messende Größen oder Parameter bedürfen unterschiedlicher Sensorkomponenten. Im Rahmen der Erfindung sind nicht nur Ausführungsformen offenbart, bei denen die Sensorkomponente selbst im Gehörgang angeordnet ist, sondern auch Ausführungsformen, bei denen sich die Sensorkomponente außerhalb des Gehörgangs befindet, es wird aber die im Gehörgang gewonnene physikalische Messgröße mit der Sensorkomponente außerhalb des Gehörgangs in Verbindung gebracht. So kann Licht beispielsweise durch LEDs außerhalb des Gehörgangs, beispielsweise in der Mini-Auswerteeinheit hinter dem Ohr erzeugt und in den Gehörgang mittels Lichtleiter fortgeleitet werden oder umgekehrt Licht aus dem Gehörgang via Lichtleiter an einen Lichtempfänger außerhalb des Gehörgangs geleitet werden. Das Prinzip der Erfindung bleibt davon unberührt, wo die optischen Komponenten angeordnet sind, der Ort, an dem die gewebsoptische Messstrecke besteht, ist der Gehörgang.

Welche Sensorkomponenten, nun im Einzelnen betrieben werden, für welche Messgröße auch immer, es ist darauf zu achten, dass die Verlustleistung im äußeren Gehörgang nicht so groß wird, dass an irgendeiner Stelle Temperaturen über 42°C auftreten, um auch bei längerfristiger Erwärmung

Gewebsschädigungen zu vermeiden. Als Richtgröße, die nicht überschritten werden sollte, hat sich in eigenen Messungen eine Verlustleistung von etwa 30 mW cw ergeben für lokal eng fokussierte Situationen, wie sie sich z.B. bei der Verwendung von LEDs für die Pulsoximetrie im bzw. am Gewebe ergeben.

Erfindungsgemäß lassen sich im äußeren Gehörgang auch optisch zugängliche Parameter bestimmen. Zur erfindungsgemäßen Messung fotometrischer und optisch-plethysmografischer Größen, z.B. Pulsoximetrie wird das subcutane Gewebe des äußeren Gehörgangs durchstrahlt. Dabei ist vorteilhaft, dass das Gewebe des äußeren Gehörgangs eine ausreichend hohe Perfusion aufweist, wodurch hohe Modulationstiefen entstehen. Der äußere Gehörgang wird quasi als Küvette mit definierter Schichttiefe angesehen, wobei die Schichttiefe dem Nettolichtweg entspricht. Der äußere Gehörgang wird ferner als Küvette für Stoffe angesehen, die im Blut des umgebenden Gewebes gelöst sind.

Bei der Gewebsphotometrie / Pulsoximetrie wird bekanntermaßen die Absorption von Licht, insbesondere die zeitlich veränderliche Absorption von Licht bzw. die Modulation in einem bestimmten Frequenzbereich beispielsweise 0 bis 0,5 Hz sowie 0,5 bis 10 Hz ermittelt, die im Zusammenhang mit der kleinsten bzw. größten zu beobachtenden Pulsfrequenz bzw. Atemfrequenz, und den Blutdruck und andere Größen des zu untersuchenden Organismus steht.

Der Lichtfluss im Gewebe kommt insbesondere durch verschiedene optische Wirkungen wie Streuung, Absorption, Beugung u. a. an Grenzflächen zwischen den unterschiedlichen Bestandteilen des lebenden Gewebes, zustande, wobei "Transmission" und "Reflexion" vereinfachende Begriffe sind, die sich eher auf ein mikroskopisch-geometrisches Verhalten des Lichts relativ zu Sender und Empfänger beziehen.

So wird bei der optischen Plethysmographie die Absorption von Licht, insbesondere die Modulation von Licht in zwei unterschiedlichen Frequenzspektren herangezogen:

❖ der sog. pulsatile Anteil, das **Wechsellichtspektrum**, auch als AC bezeichnet, im Frequenzbereich der Herzfrequenz, der arteriellen Pulsation, also von ca. 30 bis 240 bpm, entsprechend 0,5 bis 4 Hz (maximaler Bereich) oder von ca. 40 bis 150 bpm, entsprechend 0,67 bis 2.5 Hz (normaler Bereich) bzw. auch im Frequenzbereich der Atemfrequenz von 5-20 pro Minute und

❖ der sog. konstante, nicht-pulsatile Anteil, das **Gleichlichtspektrum,** auch als DC bezeichnet, im Frequenzbereich von unter 0,5 Hz, wobei sich "konstant" und "nicht-pulsatil" in Relation zur Herzfrequenz gemeint ist oder sogar relativ zur Atemfrequenz.

Die Division von AC durch DC ergibt die Modulationstiefe MD für das monochromatische Licht bzw. für das Lichtspektrum :

$$MD_{\lambda_x} = \frac{AC_{\lambda_x}}{DC_{\lambda_x}},$$

Damit lassen sich unter anderem die Pulsfrequenz und die Atemfrequenz bestimmen, gegebenenfalls auch der Blutdruck und andere Parameter.

Wird eine optische Plethysmografie in zwei oder mehr Spektralbereichen (λ1, λ2...λn) durchgeführt und werden die jeweiligen Modulationstiefen (pulsatiler Anteil, AC, zum nicht-pulsatilen Anteil, DC) z. B. bei 730 nm und 880 nm zueinander in Beziehung gesetzt, so ergibt sich aus den jeweiligen Modulationstiefen eine Variable Omega, englisch: Ratio,

$$\Omega = \frac{MD_{\lambda_1}}{MD_{\lambda_2}}, \qquad \Omega = \frac{\left(\frac{AC}{DC}\right)_{\lambda_1}}{\left(\frac{AC}{DC}\right)_{\lambda_2}}$$

die in weiten Bereichen unabhängig ist von Einflussfaktoren wie der Schichtdicke des durchstrahlten Gewebes oder der Intensität der Lichtemitter usw. und mit deren Hilfe sich die arterielle Sauerstoffsättigung ermitteln lässt, gegebenenfalls auch die venöse Sauerstoffsättigung, eventuell auch die arteriell-venöse Sättigungsdifferenz.

Der spektroskopische Hintergrund ist, dass menschliches Hämoglobin im Wesentlichen in zwei Zuständen vorkommt, nämlich oxigeniert, Hämoglobin $HB_{ox}$, und desoxigeniert, $HB_{red}$, wenn man von toxisch veränderten bzw. genetisch aberranten Hämoglobin-Fraktionen absieht.

$HB_{ox}$ und $HB_{red}$ weisen unterschiedliche spezifische spektrale Absorptionsverläufe auf, von denen sich insbesondere der Bereich zwischen 600 nm und 1000 nm für Zwecke der optischen Plethysmografie nutzen lässt. Dazu misst man die Absorption des Lichts durch die beiden Hämoglobinfraktionen (oxigeniert und desoxigeniert) in zwei verschiedenen Spektralbereichen, genauer man misst die Intensität des Lichts nach der Passsage durch Gewebe, in dem Arteriolen pulsieren, in denen Blut mit den beiden Hämoglobinfraktionen fließt.

Zur erfindungsgemäßen Messung fotometrischer und optisch-plethysmografischer Größen kommen wiederum die erfindungsgemäßen Komponenten wie Sensorträger, Sensorträgerpositionierungselement sowie die Mini-Auswerteinheit hinter der Ohrmuschel als Grundkomponenten des Sensordesigns zur Anwendung d.h. zur Positionierung, Stellung, Anordnung von Lichtemitter und Lichtempfänger insbesondere zur Herstellung geeigneter Lichtwege.

Ausführungsformen fotometrischer bzw. optisch-plethysmografischer Gehörgangssensoren, also Sensoren für die Pulsoximetrie, die Plethysmografie, bzw. für die Bestimmung von Stoffkonzentrationen weisen folgende wesentliche, vorteilhafte Merkmale hinsichtlich des Sensordesigns auf:

✳ Es wird ein Lichtweg durch die Wand des äußeren Gehörgangs, d.h. durch das Gewebe der Gehörgangswand erzeugt. Dazu wird das Licht, das von mindestens einem, meist mehreren Lichtemittern erzeugt wird, ins Gewebe des Gehörgangs eingestrahlt und tritt dann an anderer Stelle wieder aus und wird von einem Lichtempfänger detektiert. So ist eine Absorption von Licht im Gewebe gewährleistet, nur so können die optischen Eigenschaften des Gewebes bzw. des im Gewebe befindlichen Blutes bzw. von im Blut befindlichen Stoffen bestimmt werden.

✳ Essentiell ist die Vermeidung von Shuntlicht, da dies zu Messfehlern führt. Erfindungsgemäß eignen sich insbesondere lichtundurchlässige Scheiben oder Schirmchen zur Blockierung von Shuntlicht, die sich im Gehörgang aufspannen und so Lichtemitter und Lichtempfänger in zwei optisch voneinander getrennte Halbräume teilen. Licht kann so nur durch das Gewebe den Lichtempfänger erreichen. Besonders vorteilhaft ist, wenn der Sensorträger selbst zur Unterdrückung von Shuntlicht im weiteren Sinn dient, d.h. ein oder mehrere lichtundurchlässige Sensorträger sperren den direkten Lichtweg. Schon die Anordnung des Lichtemitters auf der einen Seite und Anordnung des Lichtempfängers auf der anderen Seite eines optisch undurchlässigen schirmchen- oder scheibenförmigen Sensorträgers unterbindet den direkten Lichtweg. Werden mehrere Sensorträger gestaffelt kann die Sicherheit der Unterdrückung von Shuntlicht noch verbessert werden.

✳ Um ein gutes fotometrisches bzw. optisch-plethysmografisches Signal zu erhalten, insbesondere eine hohe Modulationstiefe, ist es wichtig, dass das Licht auf seinem Weg von den Lichtemittern zum Lichtempfänger einen möglichst langen Weg im Gewebe zurücklegt, dass also der Nettolichtweg möglichst lang ist, idealerweise sogar länger als der längste geometrische Lichtweg.

✳ Lichtemitter und Lichtempfänger werden vorzugsweise nach außen orientiert und einander gegenüber angeordnet, d.h. 180° bezogen auf einen kreisförmigen, beispielsweise scheiben- oder schirmchenförmigen Sensorträger. Die beiden optischen Komponenten sind so maximal gesperrt angeordnet. Das Licht wird so in die Haut des Gehörgangs eingestrahlt und tritt auf der gegenüberliegenden Seite wieder aus, nachdem es einen Nettolichtweg etwa in Form eines Halbkreises durch das Gewebe des Gehörgangs zurückgelegt hat, genauer: zwei achsensymmetrische Halbkreise, wobei "Achse" die Verbindung zwischen Lichtemitter und Lichtempfänger bezeichnen soll. Insbesondere entsteht in 180°-Stellung durch die Symmetrie die maximale Entfernung, d.h. der längste Nettolichtweg und damit auch das beste optisch plethysmografische Signal und vor allem die höchste Modulationstiefe - echte Transmissions-Pulsoximetrie im engsten Sinne. Auch eine Anordnung, bei der Lichtemitter und Lichtempfänger einander nicht exakt gegenüber liegen (Rotation in der Sensorträger-Ebene: 90° bis 180°), ist erfolgreich. Dabei überwiegt die Lichtintensität im kürzeren Lichtweg, d.h. der kürzere Kreisbogen und somit die lichtstärkere Teilkomponente dominiert das plethysmografische Signal. Ein solcher, nichtmaximaler Lichtweg bietet sich an, wenn der Lichtweg im Halbkreis zu lang wäre, um mit vertretbarem Aufwand durchstrahlt zu werden. Allerdings beinhaltet eine Verkürzung des Lichtwegs die Gefahr von Shuntlicht.

Unter Umständen reicht *ein* Sensorträger nicht aus, um das Shuntlicht vollständig zu blockieren. Es werden also in einer weiteren erfindungsgemäßen Ausgestaltung zwei oder mehr Sensorträger hintereinander auf dem Sensorträgerpositionierungselement aufgebracht. Lichtemitter und Lichtempfänger können, wie für *einen* Sensorträger beschrieben, verschiedene Stellungen zueinander einnehmen, am besten werden sie wieder maximal gesperrt, d.h. 180° zueinander angeordnet. Als Nettolichtweg ergibt sich wieder ein Halbkreis, allerdings schräg im Gewebe verlaufend, von einem Sensorträger zur gegenüberliegenden Position auf einem anderen. Dieser Nettolichtweg ist maximal lang, maximiert also die Modulationstiefe und minimiert gleichzeitig der Shuntlichteinfluss - so wird letztlich eine echte Transmissions-Pulsoximetrie im äußeren Gehörgang erreicht.

Besonders günstig ist letztlich die Anordnung mit maximal gesperrten optischen Komponenten auf zwei oder mehr Sensorträgern, da mehrere lichtundurchlässige Sensorträger das Shuntlicht besonders zuverlässig sperren.

In einer weiteren Ausgestaltung liegen der Lichtemitter und Lichtempfänger mehr oder weniger nebeneinander (Rotation in der Sensorträger-Ebene: 0° bis 90°) - letztlich eine Reflex-Pulsoximetrie im äußeren Gehörgang. Für diese Ausgestaltung ist mehr als ein Sensorträger nötig; es wird dann von der Position auf dem einen Sensorträger zur benachbarten Position auf dem danebenliegenden Sensorträger Licht durch die Haut des äußere Gehörgang gesandt. Bei dieser Anordnung steigt die Lichtintensität, allerdings nimmt auch das Shuntlicht erheblich zu während die Modulationstiefe sinkt. Ein solcher Lichtweg bietet sich an, wenn Spektralbereiche verwendet werden, in denen die spezifische spektrale Absorption so hoch ist, dass sich nur sehr kurze Lichtwege mit vertretbarem Aufwand realisieren lassen.

✳ Zur Bestimmung fotometrischer Größen kann noch mindestens ein weiterer Lichtempfänger, optisch unmittelbar dem Lichtemitter benachbart, angeordnet werden, sodass eine zusätzliche Information über die ins Gewebe eintretende Lichtintensität vorliegt. Diese Information ist insbesondere für die Bestimmung absoluter Konzentrationen von Stoffen im Gewebe bzw. von Stoffen im Blut, das das Gewebe passiert, wichtig. Nach den Prinzipien der Fotometrie muss dazu nicht nur die Lichtintensität I nach der Passage durch das Gewebe bekannt sein, sondern

auch die Eingangslichtintensität $I_0$ d.h. die ins Gewebe eingestrahlte Lichtintensität.

✳ Günstig ist, wenn Lichtemitter und Lichtempfänger jeweils an der Peripherie eines oder mehrer Sensorträger (s) radial nach außen gewandt angeordnet werden, wobei die Richtungen der optischen Sensorkomponenten natürlich unterschiedlich sein können.

✳ Eine alternative Lichtwegführung besteht darin, das Licht einfach in den Raum des Gehörgangs auszustrahlen, also nur der Lichtempfänger ist zum Gewebe hin gewandt bzw. liegt an ihm an. Umgekehrt kann der Empfänger Licht aus dem Raum empfangen und der Lichtemitter liegt am Gewebe an. Ein gewisser Nachteil dieser Ausführungsform ist, dass so die Nettolichtwege nicht eindeutig definiert sind.

Ein gewisser Andruck der optischen Sensorkomponenten gegen das Gewebe ist generell günstig. Dabei ist allerdings zu berücksichtigen, dass der Andruck ein Optimum hat, d.h. zu wenig Andruck oder zu viel ist gleichermaßen ungünstig. Besonders ungünstig sind wechselnde Andruckverhältnisse, so wie sie Bewegungen am Sensor erzeugen bzw. Bewegungen des Sensors relativ zum Gewebe - sie erzeugen Bewegungsartefakte. Um solche Bewegungsartefakte bestmöglich zu unterdrücken oder besser noch ganz zu vermeiden, sollte das Sensorträgerpositionierungselement möglichst wenige Bewegungen übertragen, insbesondere keine Bewegungen in axialer Richtung. Konstruktiv ließen sich Bewegungen reduzieren, indem in das Sensorträgerpositionierungselement eine Vorrichtung integriert wird, die Bewegungen absorbiert oder deren Kompensation erlaubt. Zur Reduktion axialer Bewegungsartefakte würde sich eine Vorrichtung eignen, die wie eine Art Teleskopelement das Stauch und Zugbewegungen aufnimmt. Aber auch winzige Bewegungsaufnehmer könnten Bewegungen detektieren und damit ermöglichen, dass sie signaltechnisch erkannt und unterdrückt werden.

Die Anforderungen an den Sensorträger einerseits optisch undurchlässig andererseits aber akustisch permeabel zu sein, sind nicht trivial miteinander vereinbar. Als erfindungsgemäße Lösungen werden folgende grundlegende, Konzepte vorgeschlagen: Eine erste Ausführungsform umfasst einen Sensorträger aus lichtundurchlässigem Material, erlaubt aber die Ausbreitung des Schalls im Gehörgang entweder durch seine hohe materialbedingte Schwingungsbereitschaft oder durch schall-leitende aber lichtabsorbierende Strukturen wie kleine gekrümmte Röhren oder wenige, günstig gegeneinander versetzte Aussparungen. Eine erste Ausführungsform umfasst einen Sensorträger aus lichtundurchlässigem Material ohne irgendwelche Aussparungen; die Schalleitung erfolgt dann aktiv, d.h. mittels eines winzigen Mikrophons im distalen Bereich des äußeren Gehörgangs oder in der Ohrmuschel und einem winzigen Lautsprecher in der Nähe des Trommelfells.

***Messung mechanischer Größen wie Lage, Beschleunigung und Position:***

Erfindungsgemäß werden mechanische Sensoren entweder im äußeren Gehörgang positioniert, oder in der Mini-Auswerteeinheit hinter dem Ohr, einerseits per se bzw. andererseits flankierend zu Sensoren für andere Parameter. Sensoren zur Messung mechanischer Parameter sind beispielsweise Inklinationssensoren, Beschleunigungssensoren für lineare Bewegungen oder Rotation, Sensoren für die Lage des Kopfes relativ zu anderen Körperteilen, beispielsweise Rumpf und Extremitäten, oder Positionssensoren, wie beispielsweise GPS oder ähnliches. Damit lassen sich Zustände wie körperliche Aktivität, Körperhaltungen wie Stehen, Sitzen, Liegen und deren Veränderung(en), z.B. Sturz, Sturzgefahr, Unfall, Setzen, Legen, Aufstehen detektieren, sowie körperliche Anstrengung abschätzen wie Laufen, Ausdauer, Trainingsgrad, und auch Indikatoren gewinnen für Schlaf, Schlafphasen und Schlafqualität und vieles andere mehr. Diese Informationen sind für sich alleine schon wichtig. Eine besondere Bedeutung erhalten sie im Zusammenhang mit den anderen Parametern wie z.B. Herzfrequenz, Atemfrequenz oder Sauerstoffsättigung, deren Interpretation sie erheblich verbessern und erweitern: Blutdruck bei körperlicher Arbeit, Sport, Atmung während des Schlafes, Körper(kern)temperatur bei Sport, Training und Rehabilitation usw.

Informationen über Lage und Bewegungen sind erfindungsgemäß interessant für die Interpretation von Sensordaten im medizinischen Gesamtzusammenhang. Darüber hinaus erlauben Daten über Lage und Bewegungen aber auch die Einschätzung von Störgrößen. So werden in vorteilhaften Ausführungsformen die Störgrößen gemessen und diese Informationen eingesetzt um die Nutzinformationen zu korrigieren oder um die Nutzinformationen nur dann zu verwenden, wenn die Messung störungsarm verläuft.

Störgrößen können mechanische und thermische Einflüsse sein, auch aus der unmittelbaren anatomischen Umgebung: Kauen, Gähnen, Husten, aber auch das Konsumieren warmer und kalter Speisen und Getränke. Diese Störgrößen sind aber vorübergehender Natur und können daher als "hochfrequente Signale" mit signaltechnischen Methoden aus einer kontinuierlichen Messung herausgehalten werden. Andererseits kann es auch gerade im Interesse bestimmter Anwender liegen, genau diese Einflüsse zu untersuchen.

Einige Ausführungsformen der Erfindung sind anhand der Figuren beispielhaft dargestellt.

Figur 1:     Darstellung der Sensorvorrichtung

Figur 2: Darstellung des Sensorträgers

Figur 3: Querschnitt durch den äußeren Gehörgang mit Sensorkomponente auf einfachem Sensorträger

Figur 4: Querschnitt durch den äußeren Gehörgang mit Sensorkomponente auf Doppelsensorträger

Figur 5: Prinzipdarstellung der Lichtwege im Querschnitt durch den äußeren Gehörgang

Figur 6: Darstellung von Nettolichtweg und Shuntlicht bei fotometrischer bzw. optisch-plethysmografischer Sensorik

Figur 7: Darstellung schräg/spiralförmig verlaufender Lichtwege bei einem Doppelsensorträger mit 180° Positionierung der Sensorkomponenten

Figur 8: Darstellung des mäanderförmigen Temperatursensors zur Messung der Körper(kern)temperatur bzw. mäanderförmige (EKG-)Elektrode

Figur 9: Darstellung der expansiven Manschette zur Bestimmung des Blutdrucks

Figur 10: Funktionelle Verbindung der Systemkomponenten

Figur 11: Alternative Ausführungsform eines Sensorträgers mit 2 sich selbst positionierenden Kontaktpunkten

Figur 12: Sensorvorrichtung mit alternativer Ausführungsform eines Sensorträgers mit 3 sich selbst positionierenden Kontaktpunkten

Figur 13: Prinzip-Ausführungsform eines Drucksensors zum Detektieren des Blutdrucks im äußeren Gehörgang

Figur 1 zeigt die Sensorvorrichtung 26 mit den wichtigsten Komponenten. Die Mini-Auswerteeinheit 5 hinter dem Ohr wird über das anatomisch angepasste, vorgeformte Sensorträgerpositionierungselement 3 mit dem Sensorträger 2 verbunden. Der Haltefaden 4 dient zum zusätzlichen Halt der Sensorvorrichtung 26 in der Ohrmuschel. Auf dem Sensorträger 2 wird die Sensorkomponente 1 z.B. ein Temperatursensor 1 befestigt und mit den Anschlussdrähten 11 verbunden. In der Mini-Auswerteeinheit 5 können sich Heiz- und/oder Kühlelemente 6, die Energiezelle 7, ein Sender 8 und ein Inklinationssensor 9 und gegebenenfalls die Auswerteelektronik befinden.

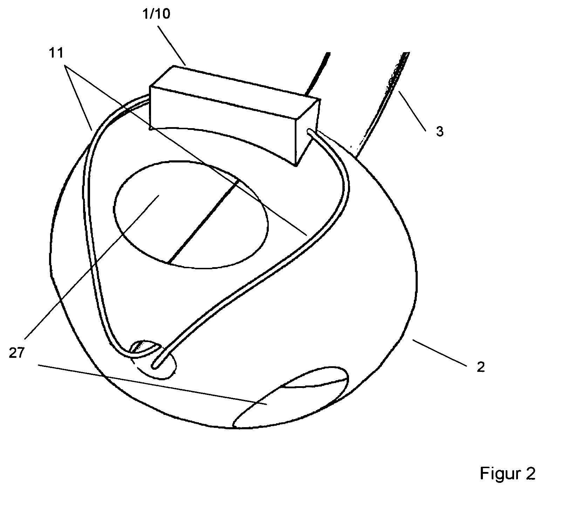

Figur 2 zeigt den Sensorträger 2 aus Figur 1 in vergrößerter Ansicht in seinem prinzipiellen Aufbau. Der Sensorträger 2 weist Aussparungen 27 auf, um die Schallausbreitung nicht zu behindern. Auf den Sensorträger 2 wird die Sensorkomponente 1 bzw.10 befestigt und mit den elektrischen Anschlussdrähten 11 verbunden. Der Sensorträger 2 ist mit dem Sensorträgerpositionierungselement 3 mechanisch verbunden. Es ist auch zu sehen, dass das Sensorträgerpositionierungselement 3 gegebenenfalls zur Aufnahme der Anschlussdrähte 11 dient.

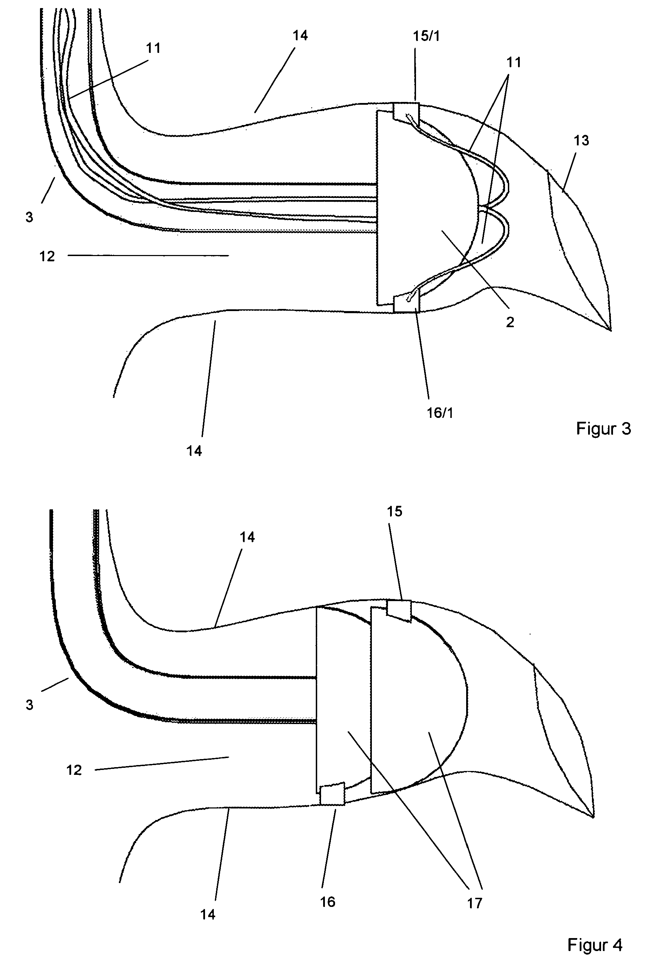

Figur 3 zeigt einen Querschnitt durch den äußeren Gehörgang 12 und einen Teil der Sensorvorrichtung 26 am Beispiel eines Pulsoximetriesensors. Mit dem Sensorträgerpositionierungselement 3 ist der Sensorträger 2 mechanisch verbunden, der die beiden Sensorkomponenten 1, z.B. die beiden optischen Komponenten Lichtemitter 15 und Lichtempfänger 16 trägt. Ferner sind die elektrischen Anschlussdrähte 11 zu sehen. Die Sensorkomponenten 1 bzw. 15 und 16 kommen an der Haut 14 zu liegen und befinden sich im äußeren Gehörgang 12 vor dem Trommelfell 13.

Figur 4 zeigt einen Querschnitt durch den äußeren Gehörgang 12 und die im Gehörgang befindlichen Komponenten Sensorträgerpositionierungselement 3, Doppelsensorträger 17, Lichtemitter 15 und Lichtempfänger 16, insbesondere für plethysmografische Sensorik, d.h. Pulsoximetrie im äußeren Gehörgang. Zu erkennen ist die optische Trennung der beiden optischen Sensorkomponenten 15 und 16 durch den Doppelsensorträger 17 zur Vermeidung von Shuntlicht, d.h. der Lichtemitter 15 kann aufgrund der optischen Trennung durch den Doppelsensorträger 17 den Lichtempfänger 16 nicht direkt, d.h. unter Umgehung eines Lichtwegs im Gewebe erreichen. Zu erkennen ist ferner, dass der Lichtemitter 15 und der Lichtempfänger 16 in axialer Richtung gegeneinander versetzt an die Haut 14 des Gehörgangs 12 gedrückt werden, wodurch eine Verlängerung des Lichtwegs und des Nettolichtwegs erreicht wird. Zudem sind der Lichtemitter 15 und der Lichtempfänger 16 um etwa 180° in radialer Richtung gegeneinander versetzt.

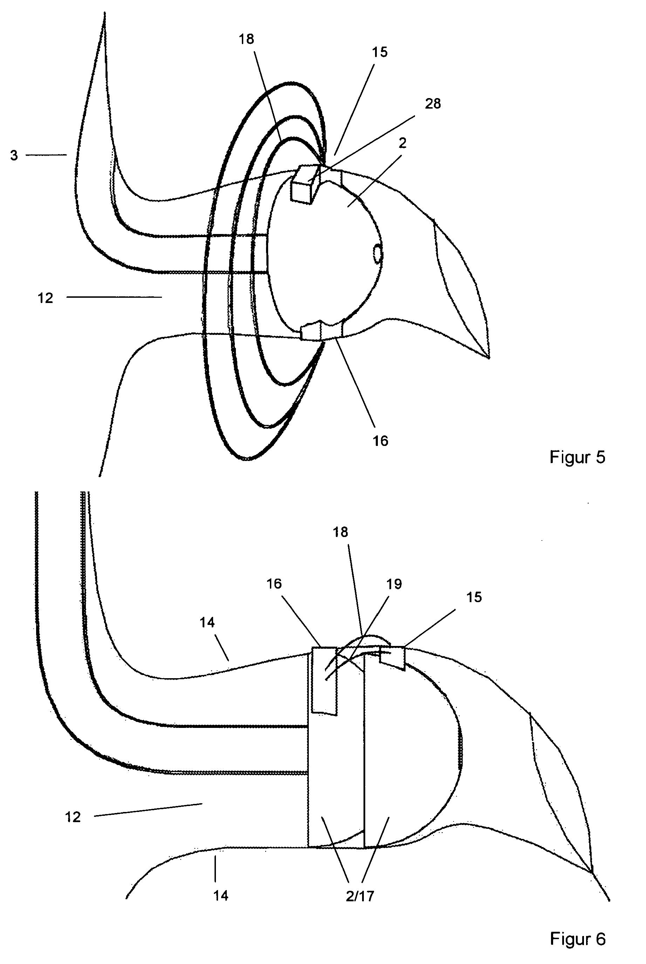

Figur 5 zeigt einen halbperspektivischen Querschnitt durch den äußeren Gehörgang 12 mit einer optischen insbesondere fotometrischen Sensorik. Es sind hier im Wesentlichen Lichtwege in der Wand des Gehörgangs dargestellt, die aufgrund von Streuung und Beugung im Gewebe gebogen zwischen dem Lichtemitter 15 und dem Lichtempfänger 16 verlaufen. Von den drei exemplarisch eingezeichneten Lichtwegen zwischen dem Lichtemitter 15 und dem Lichtempfänger 16 zeichnet sich der Nettolichtweg 18 aus, der innerste und kürzeste und relevanteste von den beliebig vielen innerhalb des Gewebes verlaufenden Lichtwegen. Zu sehen ist ferner der lichtemitternahe Lichtempfänger 28, der einen repräsentativen Teil des ins Gewebe emittierten Lichts empfängt.

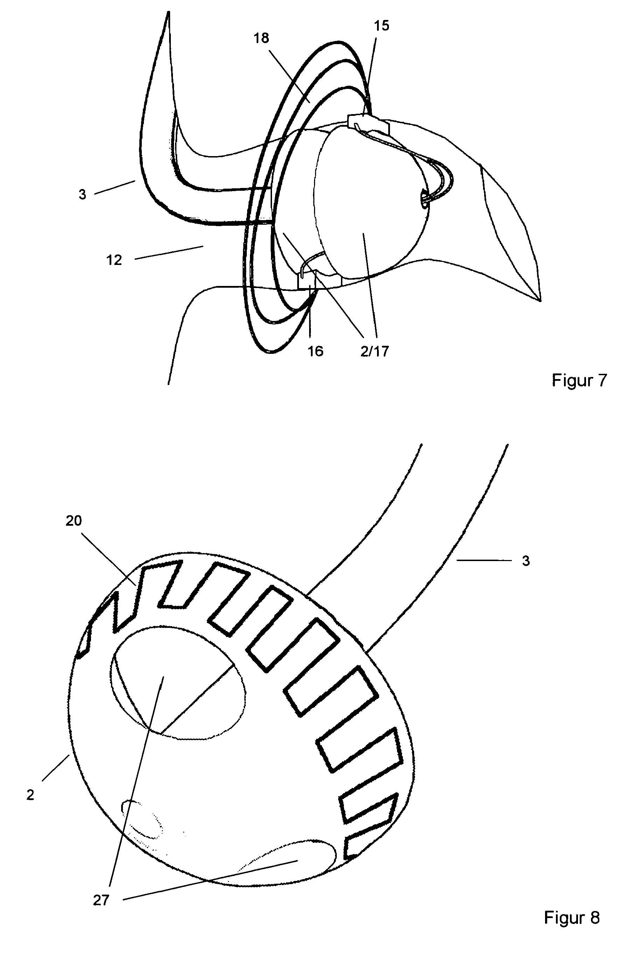

Figur 6 zeigt einen Querschnitt durch den äußeren Gehörgang 12 mit einem Doppelsensorträger 17 für plethysmografische bzw. fotometrische Sensorik. Im Gegensatz zu den Figuren 4, 5 und 7 beträgt hier der radiale Versatz-Winkel des Lichtemitters 15 und des Lichtempfängers 16 deutlich unter 90°, z.B. 0°. Durch diesen geringen radialen Versatz-Winkel zwischen dem Lichtemitter 15 und dem Lichtempfänger 16 ist einerseits der Nettolichtweg 18, der vom Lichtemitter 15 durch Haut und Gewebe 14 des Gehörgangs 12 zum Lichtempfänger 16 verläuft relativ kurz, andererseits beruht die gesamte optische Trennung zwischen dem Lichtemitter 15 und dem Lichtempfänger 16 auf nur einem Sensorträger und ist damit eventuell nicht ganz vollständig. So kommt ein Shuntlicht 19 zustande, das vom Lichtemitter 15 noch innerhalb des Gehörgangs 12, also ohne Haut und Gewebe 14 des Gehörgangs 12 durchstrahlt zu haben, direkt zum Lichtempfänger 16 verläuft.

Figur 7 zeigt einen halbperspektivischen Querschnitt durch den äußeren Gehörgang 12 mit der plethysmografischen Sensorik, das Sensorträgerpositionierungselement 3 und den Doppelsensorträger 17 mit dem Lichtemitter 15 und dem Lichtempfänger 16. Hier wird ein Doppelsensorträger 17 für die optische Trennung von Lichtemitter 15 und Lichtempfänger 16 eingesetzt, zusätzlich zu einer nahezu maximalen radialen Sperrung / Trennung des Lichtemitters 15 und des Lichtempfängers 16. Hier ist kein Shuntlichtanteil vorhanden, bzw. er ist vernachlässigbar gering. Die eingezeichneten Lichtwege verlaufen schräg bzw. spiralförmig, unter ihnen der kürzeste und effektivste, der Nettolichtweg 18. Figur 7 zeigt außerdem einen elektrischen Anschluss auf der Innenseite eines Sensorträgers 2.

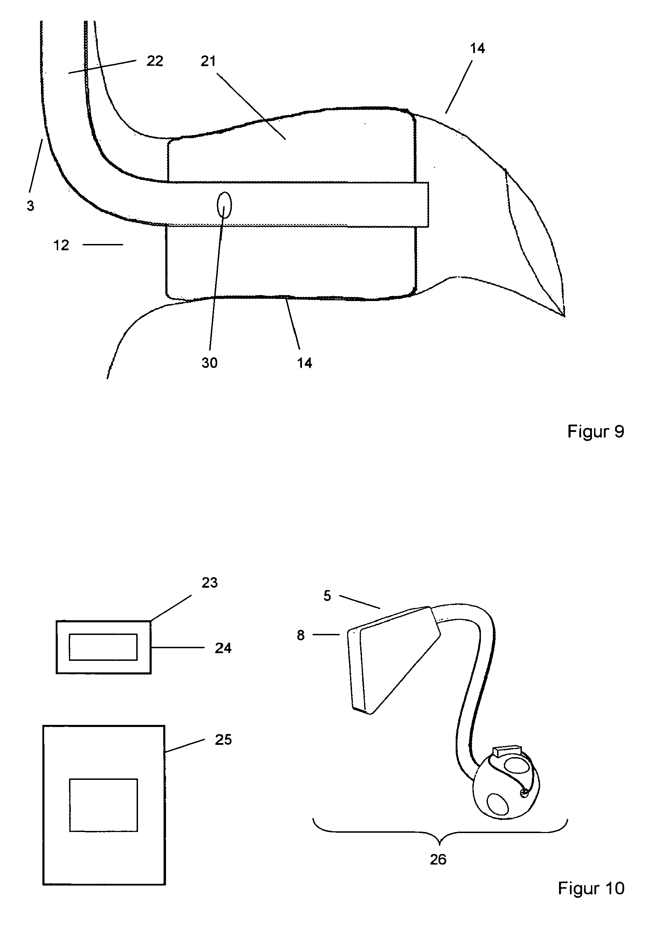

Figur 8 zeigt eine Ausführungsform eines Sensorträgers 2 mit einer alternativen Art der Temperaturmessung: Anstelle eines kleinen, quasi punktuell messenden Temperatursensors ist auf den Sensorträger 2 ist eine Widerstandsschicht 20 zur Messung der Körper(kern)temperatur mäanderförmig aufgebracht. Auf diese Weise wird die Temperatur ringförmig und über einen größeren Einzugsbereich innerhalb des Gehörgangs gemessen. Damit werden Messfehler reduziert, die dadurch zustande kommen, dass ein Teil des Sensorträgers 2 durch Faltung des Sensorträgers 2 nicht an der Wand anliegt. Bei einer ringförmigen Messung wird immer ein repräsentativer Teil der temperaturempfindlichen Schicht an der Wand des Gehörgangs anliegen; bei punktueller Messung kann gerade der messende Punkt abgehoben sein. Die Anschlussdrähte für den Temperatursensor mit Metaldampf-Mäander 20 sind nicht eingezeichnet. Der Sensorträger 2 besitzt Aussparungen 27 um die Schallausbreitung nicht zu behindern.

Figur 9 zeigt den Querschnitt des äußeren Gehörgangs 12 am Beispiel des Blutdrucksensors: Zu erkennen ist die im Gehörgang 12 expandierte, an der Haut 14 anliegende, expansive Manschette 21. Das Sensorträgerpositionierungselement 3 dient dabei als gas-/flüssigkeitführende Verbindung 22. Zu erkennen ist eine Verbindungsöffnung zwischen der expansiven Manschette und dem Sensorträgerpositionierungselement.

Figur 10 zeigt den Aufbau der gesamten Gehörgangs-Sensorik bestehend aus der in Figur 1 detaillierter beschriebenen Sensorvorrichtung 26 und die drahtlos oder drahtgebunden angeschlossenen Einheiten 23, 24 und 25 in Form von Modulen. Die Module Mini-Auswerteeinheit hinter dem Ohr 5, Anzeigeeinheit 23 bzw. Auswerteeinheit 24 und die Mobilfunkeinheit 25 können beispielsweise räumlich getrennte Einheiten sein oder auch beliebig zusammengefasst werden oder auch ganz entfallen.

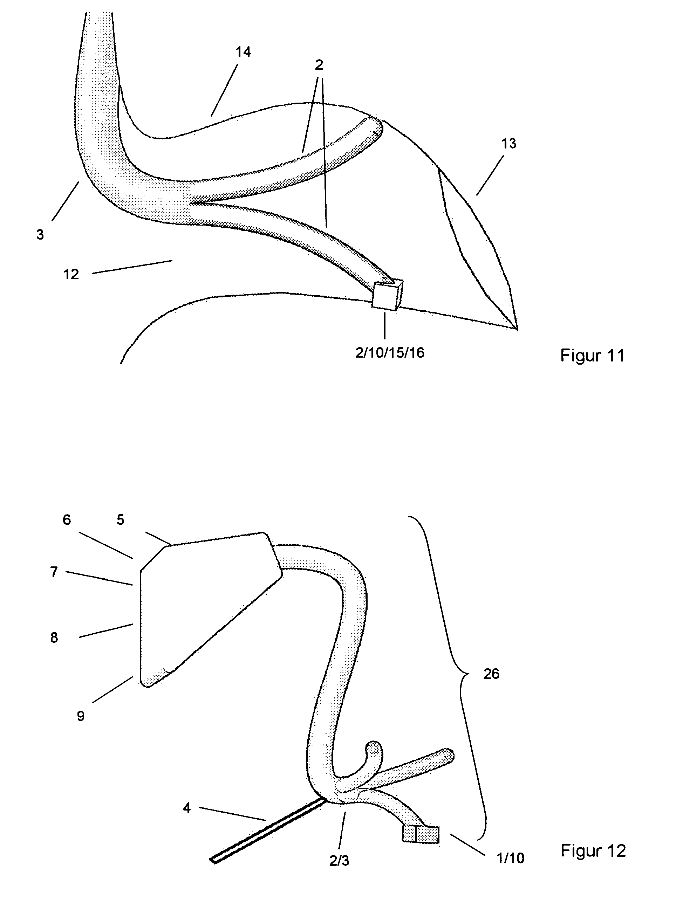

Figur 11 zeigt einen 2-poligen Sensorträger 2, d.h. eine Ausführungsform eines Sensorträgers, der mit 2 Berührungspunkten bzw. Berührungsflächen den äußeren Gehörgang 12 berührt. Damit ist in jedem Fall erzwungen, dass die Sensorkomponenten am Gehörgang anliegen. Außerdem ist gewährleistet, dass der Schall das Trommelfell nahezu ungehindert erreicht. Für Sensorik, bei der eine optische Trennung von Sensorkomponenten notwendig ist, bedarf es zusätzlicher Maßnahmen bzw. Hilfsmittel.

Figur 12 zeigt einen 3-poligen Sensorträger 2. Diese Ausführungsform eines Sensorträgers hat eine hohe zentrierende Wirkung und gewährleistet mit hoher Sicherheit, dass die Sensorkomponenten am Gehörgang anliegen. Wie in Fig. 11 ist eine Durchlässigkeit für Schall gegeben. Für Sensorik, bei der eine optische Trennung von Sensorkomponenten notwendig ist, bedarf es zusätzlicher Maßnahmen bzw. Hilfsmittel. Figur 12 zeigt ferner, dass es auch fließende Übergänge zwischen dem Sensorträger 2 und dem Sensorträgerpositionierungselement 3 gibt, d.h. es gibt Ausführungsformen, bei denen nur eine geistig abstrakte Strukturierung aber keine äußerlich erkennbare Trennung zwischen diesen beiden Komponenten existiert.

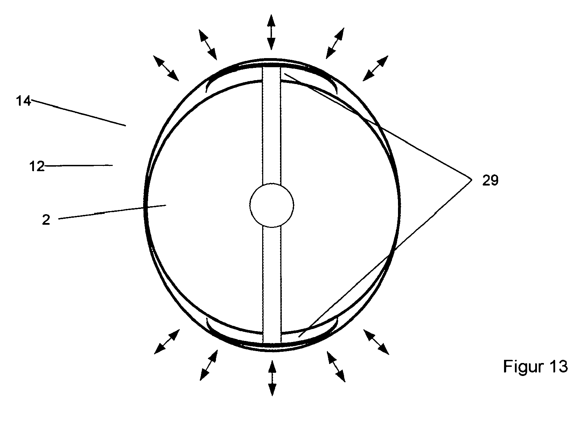

Figur 13 zeigt eine Ausführungsform eines Drucksensors, bei der anstelle einer expansiven Manschette Drucksensoren 29 verwendet werden, deren sensible Fläche klein ist, weniger Quadratmillimeter, und gebogen, d.h. der inneren Gehörgangswand angepasst. Aus Gründen der Kraftverteilung sind die Drucksensoren symmetrisch angeordnet. Drucksensor 29 kann auch als Druckgeber eingesetzt werden, d.h. er appliziert dann den Druck, der nötig ist, um den Gegendruck zu erzeugen gegen den arteriellen Druck von Seiten der Arterien und Arteriolen vorzugsweise gemäß des oszillometrischen Messprinzips.

BEZUGSZEICHENLISTE

| 1 | Sensorkomponente |
|---|---|
| 2 | Sensorträger (z. B. Einzel- Doppel- oder Mehrfachschirmchen) |
| 3 | Sensorträgerpositionierungselement |
| 4 | Haltefaden (zum Einlegen in die konvexe Helix der Ohrmuschel) |
| 5 | Mini-Auswerteeinheit hinter dem Ohr |
| 6 | Heiz- und/oder Kühlelement (in Mini-Auswerteeinheit hinter der Ohrmuschel) |
| 7 | Energiezelle (in Mini-Auswerteeinheit hinter der Ohrmuschel) |
| 8 | Sender (in Mini-Auswerteeinheit hinter der Ohrmuschel) |
| 9 | Inklinationssensor (in Mini-Auswerteeinheit hinter der Ohrmuschel) |
| 10 | Temperatursensor |
| 11 | elektrische Anschlussdrähte |
| 12 | äußerer Gehörgang |
| 13 | Trommelfell |
| 14 | Haut und Gewebe |
| 15 | Lichtemitter |
| 16 | Lichtempfänger |
| 17 | Doppelsensorträger (Doppelschirmchen) |
| 18 | Nettolichtweg |
| 19 | Shuntlicht |
| 20 | Temperatursensor mit Metalldampf-Mäander |
| 21 | expansive Manschette |
| 22 | Verbindung zum Durchführen von Gas oder Flüssigkeiten |
| 23 | Anzeigeeinheit |
| 24 | Auswerteeinheit (z.B. in verdeckter, aufgenähter Tasche) |
| 25 | Mobilfunkeinheit |
| 26 | Sensor / Sensorvorrichtung |
| 27 | Aussparung im Sensorträger für die Schallausbreitung |
| 28 | lichtemitternaher Lichtempfänger |
| 29 | Drucksensor / Druckgeber |
| 30 | Druckausgleichsloch |

**Patentansprüche**

1. Vorrichtung zur Messung mindestens eines physiologischen und/oder biochemischen und/oder bioelektrischen Parameters im Gehörgang (12) eines Menschen oder eines Tieres, umfassend

a) wenigstens eine Sensorkomponente (1), die an dem Sensorträger (2) angeordnet und/oder mit ihm verbunden ist, zur Messung mindestens eines Parameters;
b) wenigstens einen Sensorträger (2), der im Gehörgang (12) angeordnet ist;
c) ein Sensorträgerpositionierungselement (3), das die Vorrichtung im Gehörgang (12) positioniert und an einem Ende mit dem Sensorträger (2) verbunden ist,
**dadurch gekennzeichnet, dass** das Sensorträgerpositionierungselement (3) die Eindringtiefe des Sensorträgers (2) in den Gehörgang (12) bestimmt und vom Sensorträger (2) im Gehörgang (12) gehalten wird.

2. Vorrichtung nach Anspruch 1, **dadurch gekennzeichnet, dass** die Vorrichtung weiterhin eine Auswerteeinheit (5) umfasst, die zusammen mit dem Sensorträgerpositionierungselement (3)und dem Sensorträger (2) die Stabilität und den Halt der Sensorvorrichtung (26) bewirkt.

3. Vorrichtung nach einem der vorhergehenden Ansprüche, **dadurch gekennzeichnet, dass** das Sensorträgerpositionierungselement (3) und der Sensorträger (2) einstückig ausgebildet sind.

4. Vorrichtung nach einem der vorhergehenden Ansprüche, **dadurch gekennzeichnet, dass** der Parameter ausgewählt ist aus der Gruppe bestehend aus der Körpertemperatur, der Sauerstoffsättigung des Blutes, der Herzfrequenz, herzelektrischer Parameter, der Atemfrequenz, dem Blutdruck, der Konzentration von im Blut gelöster Substanzen, insbesondere der arteriellen Sauerstoffsättigung, der Konzentration von im Gewebe vorhandenen Substanzen, der körperlichen Aktivität, der Körperlage und Parametern des Schlafs und der Vigilanz.

**5.** Vorrichtung zur Bestimmung gewebsoptischer Größen, vorzugsweise der Sauerstoffsättigung im Blut, insbesondere zur Durchführung von Pulsoximetrie im Gehörgang, umfassend mindestens einen Sensorträger (2) mit einer Sensorkomponente (1) in Form wenigstens eines Lichtemitters (15) und wenigstens eines Lichtempfängers (16), **dadurch gekennzeichnet, dass** die Vorrichtung ein Mittel aufweist, das verhindert, dass Licht direkt vom Lichtemitter (15) ohne Durchstrahlung des Gewebes zum Lichtempfänger (16) gelangt.

**6.** Vorrichtung nach Anspruch 5, **dadurch gekennzeichnet, dass** die Pulsoximetrie Transmissions-Pulsoximetrie ist.

**7.** Vorrichtung nach einem der Ansprüche 5 oder 6, **dadurch gekennzeichnet, dass** das Mittel ein scheibenförmiges oder schirmförmiges Element ist, an dem der Lichtemitter (15) an einer Seite und der Lichtempfänger (16) an der anderen Seite angeordnet ist und/oder ein schirmförmiger Doppelsensorträger (17), bei dem der Lichtemitter (15) an einem Schirm und der Lichtempfänger (16) an dem anderen Schirm angeordnet ist.

**8.** Vorrichtung zur Bestimmung gewebsoptischer Größen, vorzugsweise der Sauerstoffsättigung im Blut, insbesondere zur Durchführung von Pulsoximetrie im Gehörgang, **dadurch gekennzeichnet, dass** zur Maximierung der Lichtweglänge bzw. zur Unterdrückung verkürzter Lichtwege Mittel zur stark lichtmindernden vorzugsweise lichtundurchlässigen Abdeckung von mehr als der Hälfte des halben inneren Gehörgangsumfangs im Bereich des Nettolichtwegs (18) zu beiden Seiten des Lichtempfängers (16) angewandt werden, vorzugsweise in Form eines gewölbten, schirmförmigen Sensorträgers (2) oder mehrerer solcher Sensorträger (17).

**9.** Verfahren zur Bestimmung gewebsoptischer Größen, vorzugsweise der Sauerstoffsättigung im Blut, insbesondere zur Durchführung von Pulsoximetrie im Gehörgang, **dadurch gekennzeichnet, dass** die Hilfsgröße $\Omega$ zur Bestimmung der Sauerstoffsättigung minimiert wird, beispielsweise einen Wert von maximal 0.5, typisch 0.3 bis 0.4 aufweist für humanes Oxihämoglobin und für eine Wellenlängen-Kombination von 730nm und 880nm oder für symmetrische LED-Spektren gleicher Zentralwellenlängen, und/oder korrespondierende berechenbare Omega-Werte für andere Randbedingungen.

Figur 1

1/10

11

3

2

27

Figur 2

Figur 3

Figur 4

Figur 5

Figur 6

Figur 7

Figur 8

Figur 9

Figur 10

Figur 11

Figur 12

Figur 13

14

12

2

29

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 05213099 A

- US 05662104 A